# EUROPEAN PATENT APPLICATION

(11) **EP 3 336 075 A1**
(43) Date of publication of application: **20.06.2018**
(21) Application number: 16002690.2
(22) Date of filing: 19.12.2016
(51) Int. Cl.: C07C 17/266, C07C 25/18, C07C 67/343, C07C 41/30, C07C 315/04, C07C 45/68, C07C 253/30

(54) **GOLD-CATALYZED C-C CROSS-COUPLING OF BORON- AND SILICON-CONTAINING ARYL COMPOUNDS AND ARYLDIAZONIUM COMPOUNDS BY VISIBLE-LIGHT**

(71) Applicant: Universität Heidelberg, 69117 Heidelberg (DE)
(72) Inventor: Hashmi, A Stephen K., 70565 Stuttgart (DE); Witzel, Sina, 69120 Heidelberg (DE); Xie, Jin, 69121 Heidelberg (DE); Rudolph, Matthias, 69214 Eppelheim (DE); Braje, Wilfried, 68163 Mannheim (DE)
(74) Representative: Müller-Boré & Partner Patentanwälte PartG mbB

(57) **Abstract**

The present invention relates to a method for producing (functionalized) biaryls by employing a visible-light-driven, gold-catalyzed C-C cross-coupling reaction system involving boron- and silicon-containing aryl compounds and aryldiazonium compounds. Moreover, the present invention relates to the use of such boron- and silicon-containing aryl compounds and aryldiazonium compounds, as well as related gold catalysts, in the manufacture of (functionalized) biaryls.

## Description

The present invention relates to a method for producing (functionalized) biaryls by employing a visible-light-driven, gold-catalyzed C-C cross-coupling reaction system involving boron- and silicon-containing aryl compounds and aryldiazonium compounds. Moreover, the present invention relates to the use of such boron- and silicon-containing aryl compounds and aryldiazonium compounds, as well as related gold catalysts, in the manufacture of (functionalized) biaryls.

Biaryl compounds represent an important class of synthetic building blocks, both in research and industrial environments. Accordingly, numerous synthetic approaches have been developed which require different starting materials and reaction conditions, and which allow the manufacture of a large variety of biaryl compounds for different applications.

In this context, homogenous gold catalysis has received significant attention over the last two decades. Due to the excellent carbophilic π-acidity, both gold(I) and gold(III) serve as a powerful tool to activate unsaturated C-C bonds towards nucleophilic attack without a change in oxidation state of gold during the catalytic cycle.

Besides the classical π-activation of gold catalysts without a change in oxidation state, there has been great interests in the exploration of oxidative additions of organic moieties to mononuclear and polynuclear gold(I) complexes. The aim of expanding the application of gold-mediated processes and developing novel strategies for coupling reactions is highly pursued, mimicking the classical Mⁿ/Mⁿ⁺² redox cycles of other late transition metals.

Nonetheless, different from the established palladium(0)/palladium(II) cycle, the high redox potential of the gold(I)/gold(III) redox couple requires strong external oxidants such as hypervalent iodine reagents or F⁺ donors in stoichiometric amounts. These conditions diminish one of the attractive features of gold-catalysis, mild reaction conditions and excellent functional group tolerance. In order to circumvent these harsh conditions, it has been reported to use photosensitizers and aryl radical sources (aryldiazonium or diaryl iodonium salts) combined with visible-light irradiation.

This new reactivity trend has been tentatively applied in stoichiometric organometallic chemistry, as well as catalytic C(sp²)-C(sp) bond formation reactions. During this approach one organic substituent stems from the used diazonium salt whereas the other substituent is generated by the addition of a nucleophile onto an alkyne.

Although visible light-mediated gold catalyzed C(sp²)-C(sp²) cross-couplings of using dual gold/photoredox catalysts have been reported, there are no examples of visible-light mediated, gold-catalyzed C(sp²)-C(sp²) cross-couplings without photosensitizers or an external oxidant (cf. the following Scheme 1).

### Scheme 1: Current strategies for biaryl synthesis:

### Current strategies

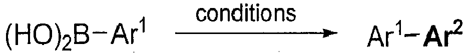

Conditions:
1) Suzuki *et al.:* Pd, **Ar²**X, base, rt or heat
2) Shi *et al*.: Au, ligand, base, Ar²N₂BF₄, rt
3) Lee *et al. and* Fouquet *et al*.: Au, Photosensitizer, **Ar²**N₂BF₄, visible light

However, all of the above-mentioned known strategies are connected to one or more disadvantages, such as that they require harsh reaction conditions, are conducted in the presence of a photosensitizer, an external oxidant or ligand, and are consequently intolerable to sensitive functional groups as substituents to the aryl groups. In addition, when using palladium as a catalyst certain functional groups such as halogens, particularly iodine, are not tolerated.

Thus, there is a need for new synthetic methods which overcome the above-mentioned disadvantages.

Accordingly, the technical problem underlying the present invention is to provide a method for effectively synthesizing (functionalized) biaryl compounds under mild reaction conditions, which does not require the presence of photosensitizers, external oxidants or ligands and which in consequence tolerates a high number of functional substituents.

Therefore, in view of the above, the present invention provides a method for manufacturing biaryl compounds, comprising the steps:
(a) providing a mixture containing a boron-containing aryl compound represented by the following Formula (i) or a silicon-containing aryl compound represented by the following Formula (ii), an aryldiazonium compound represented by the following Formula (iii) and a gold(I) catalyst in a solvent 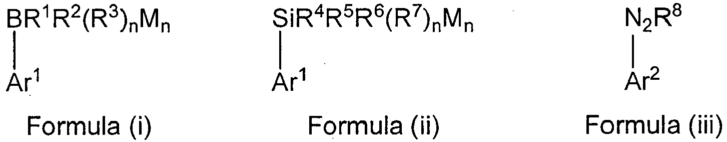 wherein
   Ar¹ and Ar² are each independently selected from a C₃-C₁₂ aryl group and a C₃-C₁₂ heteroaryl group, and each group Ar¹ and Ar² may independently contain one or more substituent(s),
   in Formula (i) R¹, R² and R³ are each independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, n represents an integer of 0 or 1, wherein two or more of R¹, R² and R³ may be bound to each other to form one or more rings and M represents a cation selected from Li, Na, K and ammonium,
   in Formula (ii) R⁴, R⁵, R⁶ and R⁷ are each independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, n represents an integer of 0, 1 or 2, wherein two or more of R⁴, R⁵, R⁶ and R⁷ may be bound to each other to form one or more rings and M represents a cation selected from Li, Na, K and ammonium,
   in Formula (iii) R⁸ represents a fluorine-containing counter-ion, and
(b) irradiating the resulting mixture with visible light.

In this context, the expressions "biaryl compound" or "biaryl" as used herein are not specifically restricted and included any compound which contains at least two aryl groups Ar¹ and Ar², wherein one aryl group Ar¹ stems from the boron- or silicon-containing aryl compound and the other aryl group Ar² stems from the aryldiazonium compound. The terms "biaryl compound" or "biaryl" explicitly also include such compounds which contain further substituents bound to the aryl groups Ar¹ and/or Ar², for example further aryl or heteroaryl groups.

The term "boron-containing aryl compound" as used herein is not specifically restricted and includes any compound which falls within the scope of Formula (i): 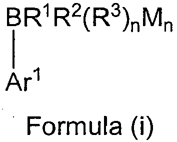 wherein R¹, R² and R³ are each independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, n represents an integer of 0 or 1, wherein two or more of R¹, R² and R³ may be bound to each other to form one or more rings and M represents a cation selected from Li, Na, K and ammonium. Moreover, both groups R¹ and R² may further form a ring, such as a 5-membered, 6-membered or 7-membered ring including the boron atom.

It is to be noted that in case the boron-containing aryl compound comprises three substituents R¹, R² and R³ (i.e. for the case of n = 1), a counter-cation M will be included to compensate for the negative charge at the boron atom. This will also be the case hereinafter, even if no charges or counter-ions are explicitly mentioned.

Moreover, the term "alkyl" used herein is not specifically restricted and may be linear, branched or cyclic and may further contain one or more substituents. According to the present invention, any substituent may include one or more heteroatoms, such as N, O and S. For example, an alkyl group containing a carbonyl group, an amine group or a thiol group will still be considered to represent a (substituted) alkyl group within the scope of the present invention. For example, the term "alkyl" includes halogenated, such as fluorinated, polyfluorinated and perfluorinated alkyl groups, and the term "alkoxy" also includes alkylesters, and the like. The same holds true for the expressions "alkenyl", "alkynyl" and "aryl" used herein, which merely require the presence of at least one C-C double bond, C-C triple bond or a delocalized π-electron system, respectively, but may further include additional substituents.

Herein, the term "ammonium" is not specifically restricted and contains any type of ammonium ion, including different grades of substitution, such as (H₄N)⁺, (H₃NR)⁺, (H₂NR₂)', (HNR₃)⁺ and (NR₄)⁺, wherein each R may, for example, represent an alkyl, alkenyl, alkynyl or aryl group.

According to a preferred embodiment, in the method of the present invention the boron-containing compound of Formula (i) is selected from a compound represented by the following Formulae (i-1) to (i-4): 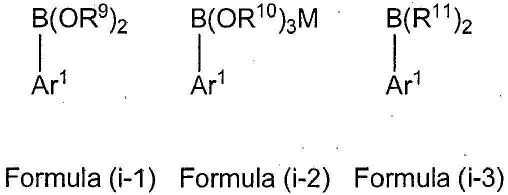 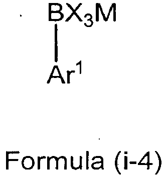 wherein
Ar¹ is as defined above,
in Formula (i-1) each R⁹ is independently selected from hydrogen, C₁-C₁₂ alkyl, C₂-C₁₂ alkenyl, C₂-C₁₂ alkynyl and C₃-C₁₂ aryl, and wherein both R⁹ may be bound to each other to form a ring,
in Formula (i-2) each R¹⁰ is independently selected from H, C₁-C₁₂ alkyl, C₂-C₁₂ alkenyl, C₂-C₁₂ alkynyl and C₃-C₁₂ aryl, wherein two or all of R¹⁰ may be bound to each other to form one or more rings and M represents a cation selected from Li, Na, K and ammonium,
in Formula (i-3) each R¹¹ is independently selected from C₁-C₁₂ alkyl, C₂-C₁₂ alkenyl, C₂-C₁₂ alkynyl and C₃-C₁₂ aryl, and wherein both R¹¹ may be bound to each other to form a ring, and
in Formula (i-4) each X is independently selected from halogen and M represents a cation selected from Li, Na, K and ammonium.

Such boron-containing aryl compounds are easily accessible as a starting material and show excellent reactivity in the method of the present invention. Moreover, the boron-containing aryl compounds as defined above are moisture and air stable and are significantly less toxic compared to classical transmetallation agents.

In a further embodiment of the method of the present invention, the boron-containing compound of Formula (i) is selected from a compound represented by the following Formulae (i-1-1) to (i-4-1): 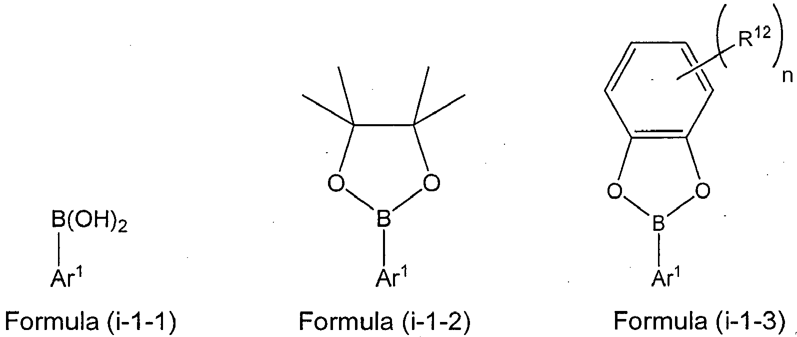 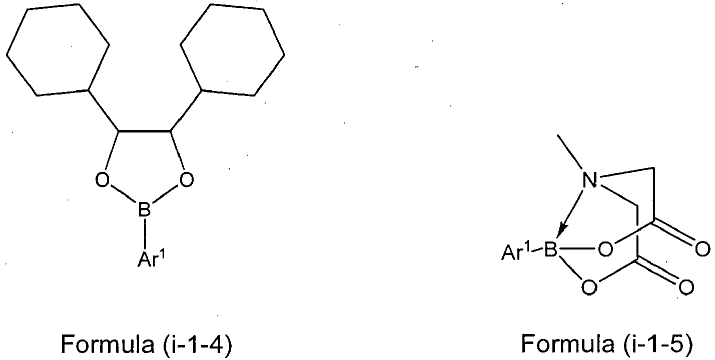 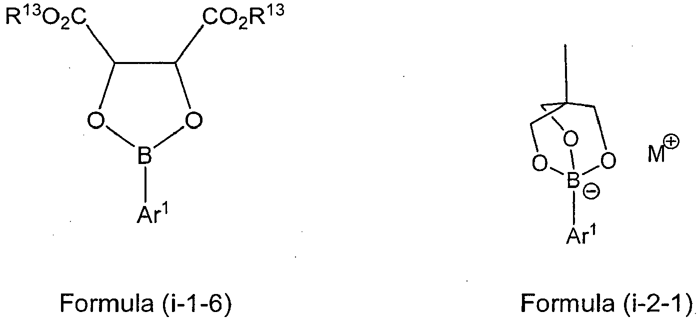 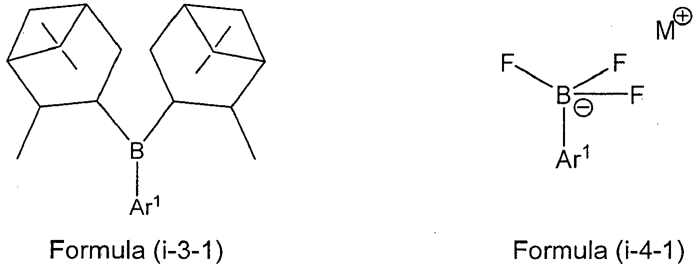 wherein
Ar¹ is as defined above,
in Formula (i-1-3) each R¹² is independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, wherein n represents an integer of 0 to 4 and one or more of R¹² may be bound to each other to form one or more rings,
in Formula (i-1-6) each R¹³ is independently selected from hydrogen, C₁-C₁₂ alkyl, C₂-C₁₂ alkenyl, C₂-C₁₂ alkynyl, and C₃-C₁₂ aryl, and wherein both R¹³ may be bound to each other to form a ring, and
in Formulae (i-2-1) and (i-4-1) M represents a cation selected from Li, Na, K and ammonium.

Alternatively, as a starting material, specific silicon-containing aryl compounds of Formula (ii) may be used in the above-defined method of the present invention. In this context, according to a further embodiment, the silicon-containing compound of Formula (ii) is selected from a compound represented by the following Formula (ii-1) to (ii-4): 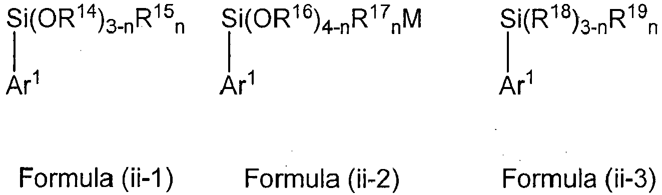 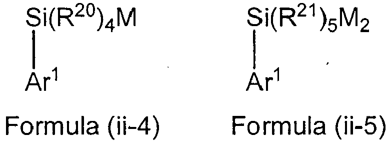 wherein
Ar¹ is as defined above,
in Formula (ii-1) each R¹⁴ is independently selected from H, C₁-C₁₂ alkyl, C₂-C₁₂ alkenyl, C₂-C₁₂ alkynyl and C₃-C₁₂ aryl, each R¹⁵ is independently selected from H, hydroxy, halogen, amino, C₁-C₁₂ alkyl, C₁-C₁₂ alkoxy, C₂-C₁₂ alkenyl, C₂-C₁₂ alkenoxy, C₂-C₁₂ alkynyl, C₂-C₁₂ alkynoxy, C₃-C₁₂ aryl and C₃-C₁₂ aryloxy, wherein two or more of R¹⁴ and R¹⁵ may be bound to each other to form one or more rings and n represents an integer of 0 to 3,
in Formula (ii-2) each R¹⁶ is independently selected from H, C₁-C₁₂ alkyl, C₂-C₁₂ alkenyl, C₂-C₁₂ alkynyl and C₃-C₁₂ aryl, each R¹⁷ is independently selected from H, hydroxy, halogen, amino, C₁-C₁₂ alkyl, C₁-C₁₂ alkoxy, C₂-C₁₂ alkenyl, C₂-C₁₂ alkenoxy, C₂-C₁₂ alkynyl, C₂-C₁₂ alkynoxy, C₃-C₁₂ aryl and C₃-C₁₂ aryloxy, wherein two or more of R¹⁶ and R¹⁷ may be bound to each other to form one or more rings and n represents an integer of 0 to 4,
in Formula (ii-3) each R¹⁸ and R¹⁹ is independently selected from H, hydroxy, halogen, amino, C₁-C₁₂ alkyl, C₂-C₁₂ alkenyl, C₂-C₁₂ alkynyl and C₃-C₁₂ aryl, and wherein two or more of R¹⁸ and R¹⁹ may be bound to each other to form one or more rings,
in Formula (ii-4) each R²⁰ is independently selected from H, hydroxy, halogen, amino, C₁-C₁₂ alkyl, C₂-C₁₂ alkenyl, C₂-C₁₂ alkynyl and C₃-C₁₂ aryl, wherein two or more of R²⁰ may be bound to each other to form one or more rings and M represents a cation selected from Li, Na, K and ammonium, and
in Formula (ii-5) each R²¹ is independently selected from H, hydroxy, halogen, amino, C₁-C₁₂ alkyl, C₂-C₁₂ alkenyl, C₂-C₁₂ alkynyl and C₃-C₁₂ aryl, wherein two or more of R²¹ may be bound to each other to form one or more rings and each M independently represents a cation selected from Li, Na, K and ammonium.

Similarly as stated for the specific case of four-valent boron-containing aryl compounds of Formula (i), it is to be noted that in case the silicon-containing aryl compound of Formula (ii) comprises four substituents R¹ to R⁴ (i.e. for the case of n = 1) or five substituents four substituents R¹ to R⁵ (i.e. for the case of n = 2), one or two counter-cations M will be included to compensate for the negative charge(s) at the silicon atom. This will also be the case hereinafter, even if no charges or counter-ions are explicitly mentioned.

For example, the aforementioned silicon-containing aryl compound of Formula (ii-3) includes silanoles (R¹⁹ = OH) of the general formula Ar¹-Si(R¹⁸)₃₋ₙ(OH)ₙ and organofluorosilanes (R¹⁹ = F) of the general formula Ar¹-Si(R¹⁸)₃₋ₙFₙ, wherein R¹⁸ is as defined above.

In a further embodiment of the present invention, in the above-defined method the silicon-containing compound of Formula (ii) is selected from a compound represented by the following Formulae (ii-1-1) to (ii-5-1): 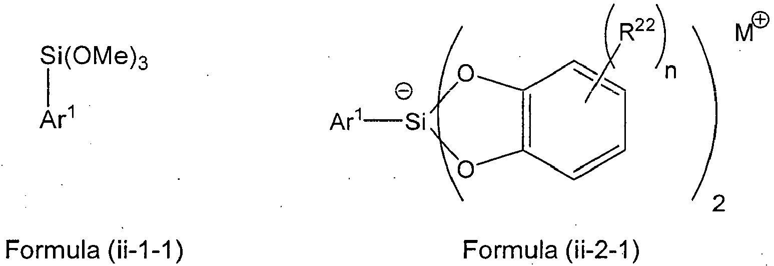 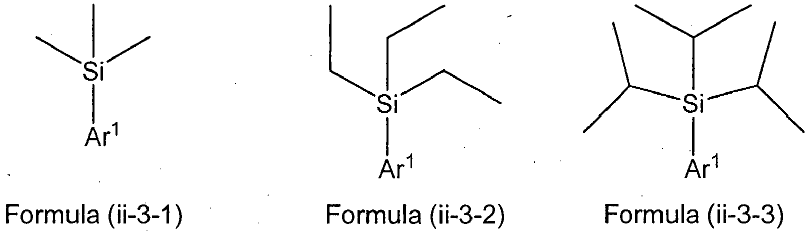 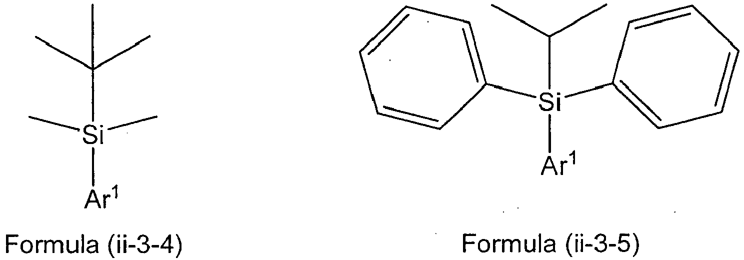 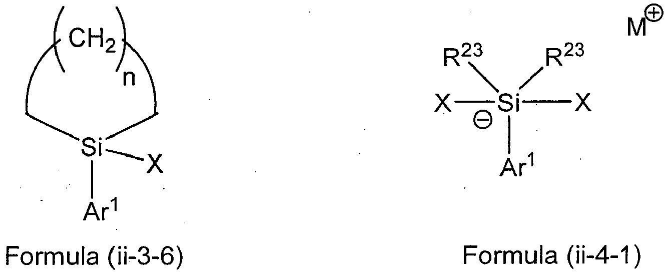 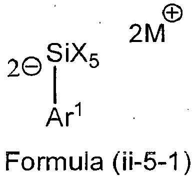 wherein
Ar¹ is as defined above,
in Formula (ii-2-1) each R²² is independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, wherein n represents an integer of 0 to 4, one or more of R²² may be bound to each other to form one or more rings and M represents a cation selected from Li, Na, K and ammonium,
in Formula (ii-3-6) X represents halogen and n represents an integer of 1 to 4,
in Formula (ii-4-1) each R²³ is independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, X represents halogen, wherein one or more of R²³ may be bound to each other to form one or more rings and M represents a cation selected from Li, Na, K and ammonium, and
in Formula (ii-5-1) X represents halogen and each M represents a cation selected from Li, Na, K and ammonium.

Depending on various factors such as desired reactivity, solubility in specific solvents, steric requirements, etc., the skilled person can readily chose suitable boron- or silicon-containing aryl compounds to be used in the method of the present invention.

The counter-ion R⁸ of the aryldiazonium compound of Formula (iii) usable in the method of the present invention contains at least one fluorine atom, since it is considered to activate the boron-containing or silicon-containing aryl compound. However, the counter-ion is not further limited and includes any fluorine-containing anion which may be effectively used in the method of the present invention, depending of the individual requirements of the reaction system, such as solubility/dissociation constant, ion strength, etc.

In this context, according to a further embodiment, in the method as defined above the group R⁸ of the aryldiazonium compound of Formula (iii) is selected from BF₄, PF₆, SbF₆, OTf, NTf₂, OSO₂C₄F₉, F, OSO₂F, BArF20, BArF24, brosylate, carborane, C(TF)₃, B(Ph)₄, Altebat, Bortebat, PFTB, and C(CF₃)₄.

According to a preferred embodiment of the method as defined above, the aryldiazonium compound is represented by one of the following Formulae (iii-1) and (iii-2):

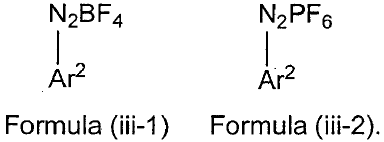

According to a further embodiment, in the method of the present invention, the aryl groups Ar¹ and Ar² are independently selected from furanyl, pyrrolyl, thiophenyl, imidazolyl, pyrazolyl, oxazolyl, isoxazolyl, thiazolyl, phenyl, pyridinyl, pyrazinyl, pyrimidinyl, pyradizinyl, benzofuranyl, indolyl, benzothiophenyl, benzimidazolyl, indazolyl, benzoxazolyl, benzisoxazolyl, benzothiazolyl, isobenzofuranyl, isoindolyl, purinyl, naphthyl, chinolinyl, chinoxalinyl and chinazolinyl.

The aryl groups Ar¹ and Ar² may be the same or different, and may be any group which contains an aromatic ring, such as a 3-membered, 5-membered or 6-membered aromatic ring. The aryl groups may be neutral or charged, such in the case of cyclopentadienyl group, and then contain a respective counter-ion.

In a specific embodiment, the aryl group may be bound to the boron or silicon atom of the boron- and silicon-containing aryl compounds (i) and (ii) directly, or may be bound thereto via another linker group, such as a vinyl group, as long as the boron and silicon atoms, respectively, are bound to a conjugated π-system.

According to the present invention, the aryl groups Ar¹ and Ar² may be substituted or unsubstituted. Since the method of the present invention is carried out under mild conditions and preferably in the absence of any photosensitizer, external oxidant or ligand, it is extremely compatible with a wide number of sensitive substituents.

Thus, in the present invention, the substituents which may be present in each of the aryl groups Ar¹ and Ar² are neither restricted in number nor type. In particular, the substituents may be independently selected from hydrogen, halogen, nitro, hydroxy, cyano, carboxyl, C₁-C₆ carboxylic acid ester, C₁-C₆ ether, C₁-C₆ aldehyde, C₁-C₆ ketone, sulfonyl, C₁-C₆ alkylsulfonyl, C₁-C₆ alkyl, C₁-C₆ haloalkyl (such as -CF₃), C₁-C₈ cycloalkyl (such as cyclopropyl), C₁-C₈ halocycloalkyl (such as difluorocyclobutyl), C₁-C₈ heterocycloalkyl (such as oxetan), C₁-C₆ alkoxy, C₁-C₆ haloalkoxy (such as -OCF₃), C₃-C₁₂ aryl, C₃-C₁₂ heteroaryl and spiro-groups (such as 2-oxa-spiro[3.3]heptane). Moreover, in each aryl group Ar¹ and Ar², there may be one, two, three, four or five substituents, which may be the same or different from each other.

Specific examples of such substituted aryl groups Ar¹/Ar² are given in the following Table 1:

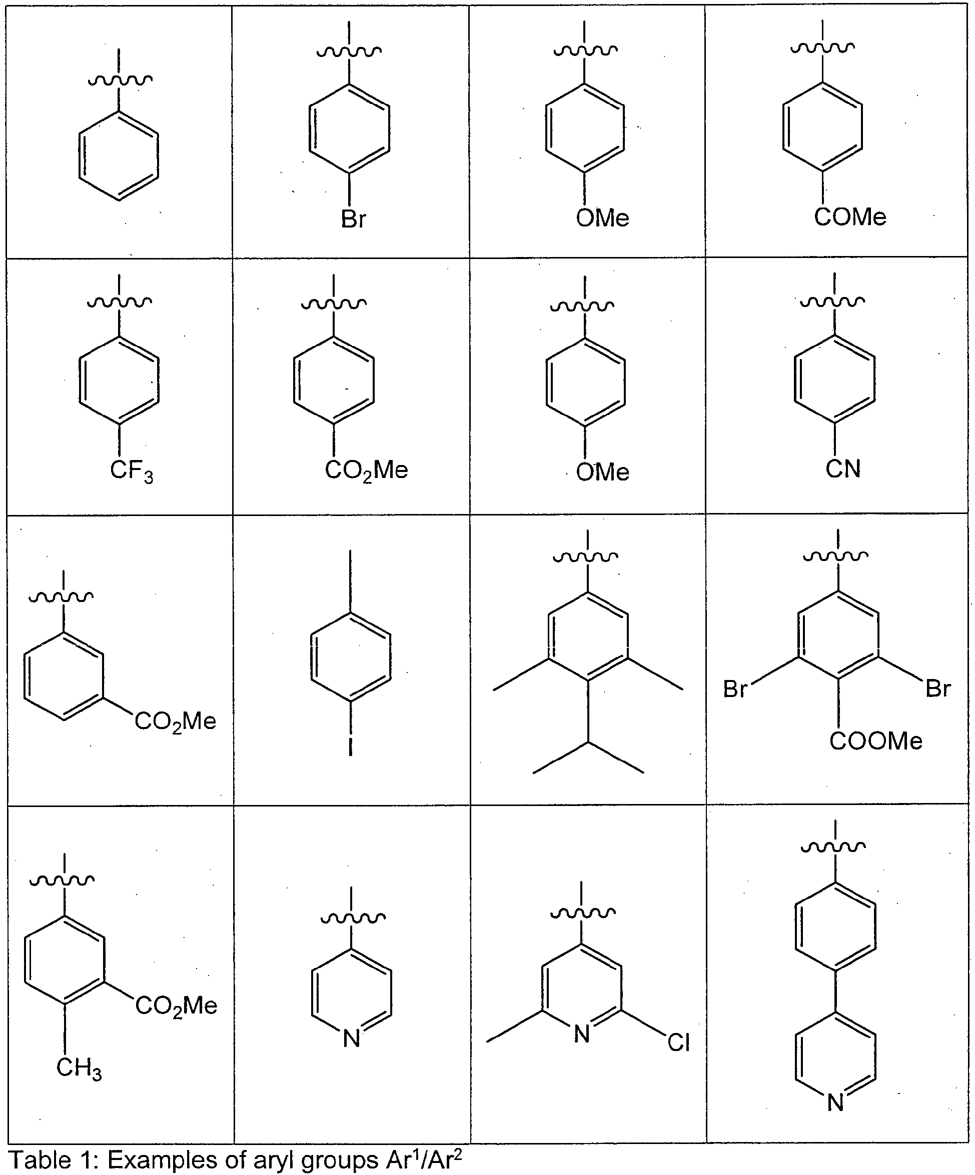

Of course, also more complex aryl groups can be used as the aryl groups Ar¹ and Ar² in the method of the present invention, such as (hetero)aryl groups which are substituted with one or more polycyclic aliphatic or aromatic substituents, etc. Due to the mild reaction conditions mentioned above the method of the present invention allows the synthesis of various biaryls despite the presence of even such complex aryl groups optionally containing further substituents. Also, the reaction mechanism underlying the method of the present invention tolerates sensitive substituents, such as iodine, which are e.g. not tolerated in classical Pd-catalyzed cross couplings.

According to the present invention, the gold(I) catalyst is not specifically restricted as long as it effectively catalyzes a visible-light induced C-C crosscoupling between the boron- or silicon-containing aryl compound and the aryldiazonium compound.

According to a further embodiment of the method as defined above, the gold(I) catalyst is selected from the group consisting of (4-CF₃-C₆H₄)₃PAuCl, Ph₃PAuNTf₂, Cy₃PAuCl, (4-Me-C₆H₄)₃PAuCl and (4-CF₃-C₆H₄)₃PAuNTf₂.

Typically, the amount of catalyst used is not specifically restricted and includes, for example, amounts in the range of 0.001 to 30 mol%, relative to the amount of the boron- or silyl-containing aryl compound/aryldiazonium compound. Further examples include ranges of 0.005 to 25 mol%, 0.01 to 20 mol% or 0.05 to 15 mol%.

Moreover, the solvent usable in the method of the present invention is not particularly restricted, as long as an effective formation of the desired biaryls can be achieved in the presence thereof. The solvent may be chosen by the skilled person in regard to desired properties, such as polarity, starting material solubility, etc.

In a further embodiment of the above-defined method, the solvent is selected from the group consisting of MeOH, EtOH, MeCN. The solvent may be a single solvent or a solvent mixture of two or more solvents. Preferably, the solvent is MeOH or contains at least 50%, at least 60% or at least 75% MeOH by volume.

As mentioned above, the method of the present invention advantageously allows the synthesis of biaryls via a visible-light driven, gold catalyzed C-C crosscoupling without requiring any photosensitizers, external oxidants or ligands.

A further embodiment of the present invention therefore relates to the method as defined above, wherein the method is carried out in the absence of a photosensitizer and external oxidant.

In a further embodiment, irradiation in step (b) is carried out at a temperature of 0 to 60°C for a duration of 10 min. to 24 hours. In a preferred embodiment, the irradiation step (b) is carried out at a temperature range of 0° to 50°C or even more preferred, a temperature range of 0 to 30°C. In particular, the reaction temperature is of secondary importance for the reaction kinetics, which is mainly influenced by the type and intensity of the irradiated light. Consequently, in a preferred embodiment, the method of the present invention is carried out at room temperature.

A further embodiment relates to the method as defined above, wherein the visible light has a maximum peak wavelength λₘₐₓ in the range of 400 to 520 nm, for example in a range of 410 to 500 nm, a range of 420 to 490 nm or a range of 440 to 480 nm. Preferred examples of the maximum peak wavelength λₘₐₓ are within the range of 460 to 475 nm, such as 470 nm, as e.g. created by blue LEDs. Wavelength and intensity of the irradiated light can be chosen in accordance with the gold(I) catalyst used in the method of the present invention and in view of optimized reaction performance.

According to a specifically preferred embodiment of the present invention, the method as defined above is carried out using a boron-containing aryl compound of Formula (i-1-1), an aryldiazonium tetrafluoroborate compound of Formula (iii-1). In this specific embodiment, (4-CF₃-C₆H₄)₃PAuCl is preferably used as the gold(I) catalyst, and the solvent is preferably methanol (MeOH).

In another embodiment, the method of the present invention may comprise a further step (c) of isolating the biaryl product from the reaction mixture. Procedures for isolating the biaryl product can readily be chosen by a skilled person and are known in the state of the art.

A further aspect of the present invention relates to the use of a boron-containing compound of Formula represented by the following Formulae (i-1-1) to (i-4-1): 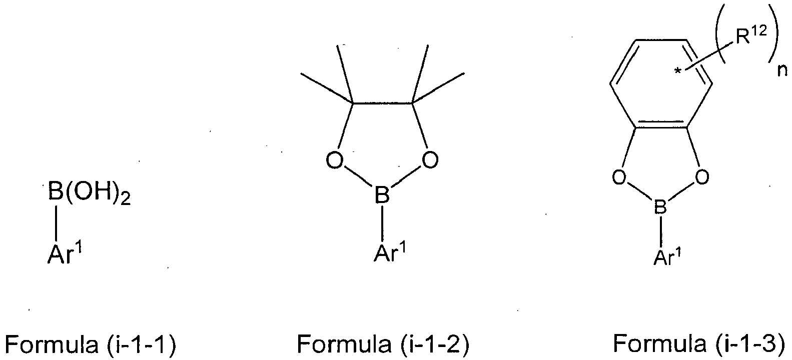 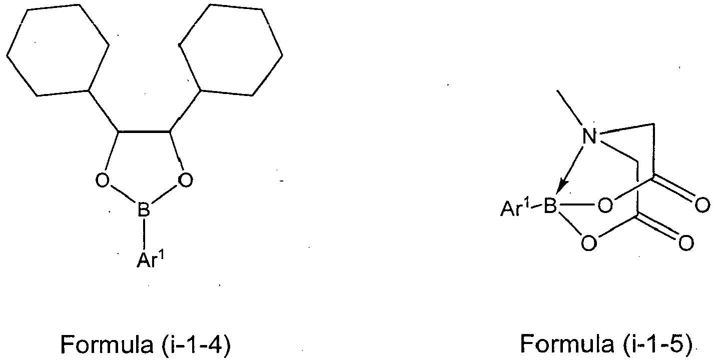 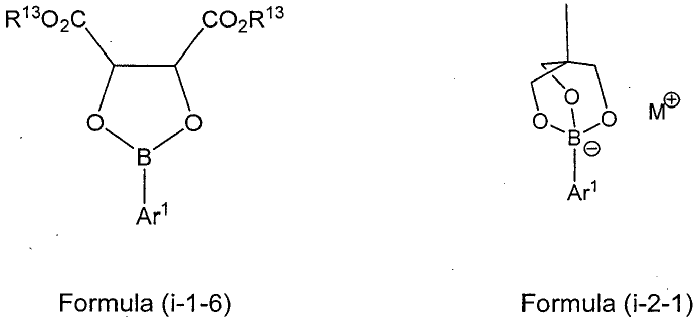 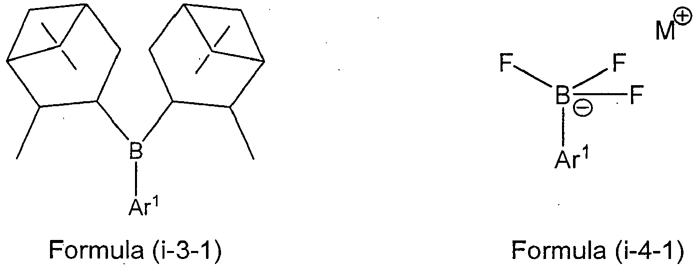 wherein
Ar¹, R¹², R¹³, n and M are as defined above,
in the manufacture of functionalized biaryls.

An even further aspect of the present invention relates to a use of a silicon-containing compound represented by the following Formulae (i-1-1) to (i-4-1): 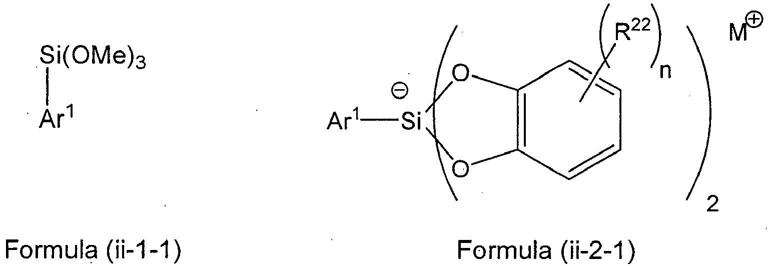 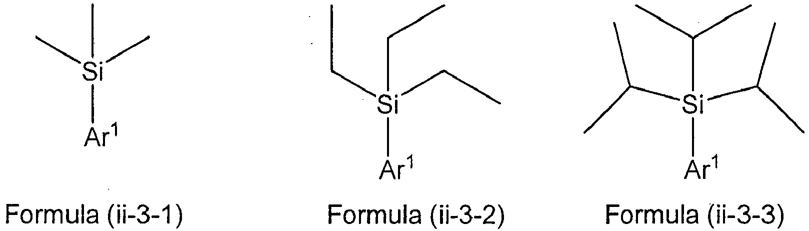 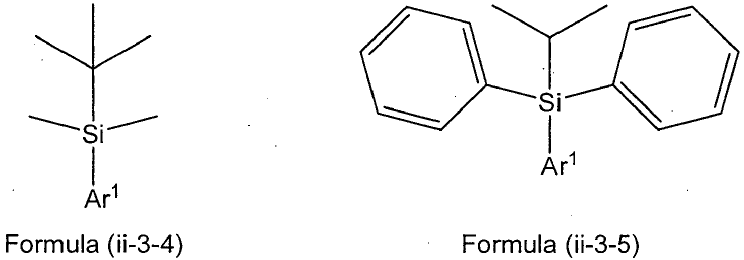 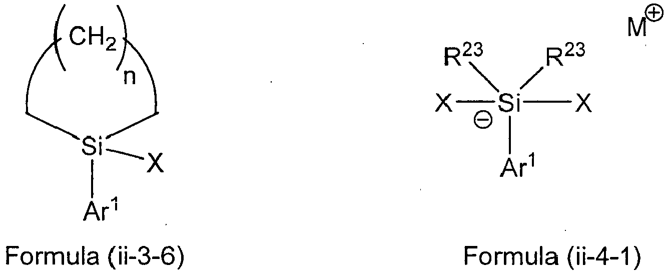 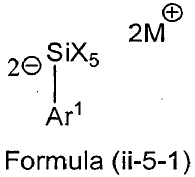 wherein
Ar¹, R²², R²³, X, n and M are defined above,
in the manufacture of functionalized biaryls.
Yet another aspect of the present invention relates to the use of (4-CF₃-C₆H₄)₃PAuCl, Ph₃PAuNTf₂, Cy₃PAuCl, (4-Me-C₆H₄)₃PAuCl or (4-CF₃-C₆H₄)₃PAuTf₂ as a catalyst in the manufacture of optionally functionalized biaryls.

The present invention provides a novel and advantageous method for the synthesis of biaryls. The method of the present invention is carried out under very mild conditions, and in the absence of a photosensitizer or and external oxidant or ligand, thus making it tolerable to a variety of sensitive functional groups. It is therefore surprisingly possible to provide easy access to a high number of sensitive or complex biaryls in good to excellent yields and purity.

### The figures show:

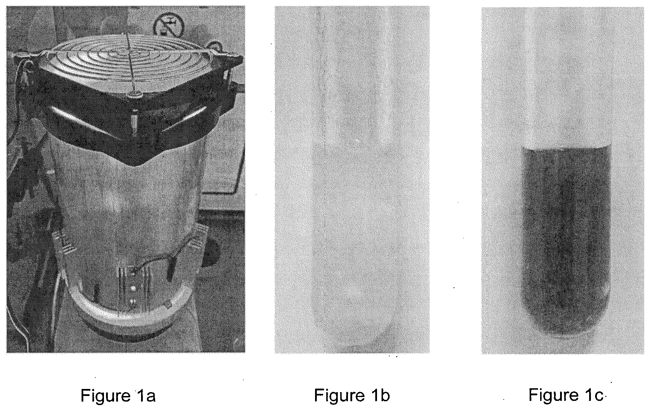

Figure 1 shows Left: The photoreactor which is equipped with 29 W LED stripes (λmax = 470 nm) and a fan on top to keep the reactor in a temperature range of 0 to 60°C, preferably around room temperature, during the reaction processes. Right: Reaction mixture before irradiation with blue LEDs (left), reaction mixture after irradiation with blue LEDs for 16 h (right).
Figure 2 shows a graph wherein the slope equals the quantum yield (Φ) of the photoreaction. Φ = 0.3021 (=30.2%).

The following examples are intended to further illustrate the present invention. However, the present invention is not limited to these specific examples.

Examples:

### 1. General information

All commercially available chemicals were purchased from suppliers (ABCR, Acros, Alfa Aesar, Chempur, Merck and Sigma Aldrich) or obtained from the chemical store of the University of Heidelberg and were used without further purifications. Dry solvents were dispensed from solvent purification system MB SPS-800-Benchtop. Deuterated solvents were supplied from Euriso-Top and used as received. The NMR spectra, if not noted otherwise, were recorded at room temperature on the following spectrometers: Bruker Avance III 300 (300 MHz), Bruker Avance DRX 300 (300 MHz), Bruker Avance III 400 (400 MHz), Bruker Avance III 500 (500 MHz), Bruker Avance III 600 (600 MHz) or Fourier 300 (300 MHz). Chemical shifts δ are quoted in parts per million (ppm) and coupling constants J in hertz (Hz). ¹H and ¹³C spectra are calibrated in relation to the deuterated solvents, namely CDCl₃ (7.26 ppm; 77.16 ppm). ³¹P spectra were calibrated in relation to the reference measurement of phosphoric acid (0.00 ppm). ¹⁹F spectra were calibrated in relation to the reference measurement of 1,2-difluorobenze (-139 ppm). The following abbreviations were used to indicate the signal multiplicity: for the ¹H NMR spectra: s (singlet), d (doublet), t (triplet), q (quartet), quint (quintet), sext (sextet), sept (septet), m (multiplet), as well as their combinations; for the ¹³C NMR spectra: s (quaternary carbon), d (tertiary carbon (CH)), t (secondary carbon (CH₂)) and q (primary carbon (CH₃)). All the ¹³C NMR spectra were measured with ¹H-decoupling and were interpreted with the help of DEPT135, ¹H, ¹H-COSY and HMBC. All spectra were integrated and processed using TopSpin 3.5 software. Mass spectra (MS and HRMS) were determined in the chemistry department of the University Heidelberg under the direction of Dr. J. Gross. EI⁺-spectra were measured on a JOEL JMS-700 spectrometer. For ESI⁺-spectra a Bruker ApexQu FT-ICR-MS spectrometer was applied. Gas chromatography / Mass Spectroscopy (GC MS) were carried out on two different systems: 1. HP 5972 Mass Selective Detector, coupled with a HP 5890 SERIES II plus Gas Chromatograph. 2. Agilent 5975C Mass Selective Detector, coupled with an Agilent 7890A Gas Chromatograph. In both cases, as a capillary column, an OPTIMA 5 cross-linked Methyl Silicone column (30 m x, 0.32 mm, 0.25 mm) was employed, and helium was used as the carrier gas. Flash Column Chromatography was accomplished using Silica gel 60 (0.04 - 0.063 mm / 230 - 400 mesh ASTM) purchased from Macherey-Nagel as stationary phase. As eluents the respectively mentioned proportions of petroleum ether (PE) and ethyl acetate (EA) were used. Analytical Thin Layer Chromatography (TLC) was carried out on precoated Macherey-Nagel POLYGRAM^{®} SIL G/UV254 or Merck TLC Silical Gel 60 F254 aluminium sheets. Detection was accomplished using UV-light (254 nm), KMnO₄ (in 1.5M Na₂CO₃ (aq.)), molybdatophosphoric acid (5 % in ethanol), vanillin / H₂SO₄ (in ethanol) or anisaldehyde / HOAc (in ethanol).

The aryldiazonium tetrafluoroborates were synthesized according to a modified procedure reported by König et al. (D. P. Hari, P. Schroll, B. König, J. Am. Chem. Soc. 2012, 134, 2968-2961). The neutral gold complexes were prepared after a procedure published by Hashmi et al. (L. Huang, M. Rudolph, F. Rominger, A. S. K. Hashmi, Angew. Chem. Int. Ed. 2016, 55, 4808-4813) and the synthesis of the cationic gold complexes proceeded after a modification of a literature report by Ogawa et al. (T. Tamai, K. Fujiwara, S. Higashimae, A. Nomoto, A. Ogawa, Org. Lett. 2016, 18, 2114-2117).

### 2. General procedures

### 2.1 General procedure for the synthesis of aryldiazonium tetrafluoroborate (GP1)

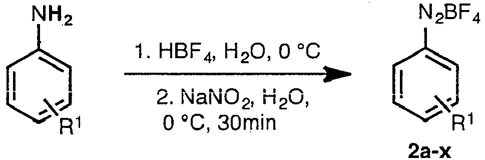

The corresponding aniline (10 mmol, 1.0 equiv.) was dissolved in a mixture of water (3.5 mL) and 3.5 mL of a 48 wt.% tetrafluoroboric acid solution in H₂O. After cooling to 0 °C an aqueous solution of sodium nitrite (690 mg, 10 mmol, 1.0 equiv., in 1.0 mL H₂O) was added dropwise over a course of 10 min. The reaction mixture was stirred for 30 min and the resulting precipitate was collected by filtration. The crude product was purified by dissolving in a minimum amount of acetone. The product was precipitated by addition of Et₂O, which was again collected by filtration. For further purification this can be repeated several times. After drying under high vacuum the corresponding diazonium tetrafluoroborate was obtained and stored at -20 °C.

### 2.2 General procedure for the synthesis of gold complexes (GP2)

DMSAuCl (1.0 equiv.) was dissolved in DCM (10 mol/l) and the corresponding ligand (1.0 equiv.) was added. After stirring for 2 hours at room temperature in the dark, the solvent was removed under reduced pressure at room temperature in the dark. The crude product was purified by dissolving in a minimum amount of DCM and the gold complex was precipitated by addition of n-pentane or PE. After filtration and drying under high vacuum in the dark, the corresponding gold complex was obtained and stored at -20 °C.

### 2.3 General procedure for the synthesis of cationic gold complexes (GP3)

The corresponding gold complex of GP2 (1.0 equiv.) was dissolved in DCM (40 mmol/l) and AgNTf₂ (1.0 equiv.) was added. After the reaction mixture was stirred for 15 min at room temperature, the precipitated AgCl was removed by filtration through a Celite Pad. The filtrate was concentrated under reduced pressure and the obtained cationic gold complex was dried under high vacuum.

### 2.4 General procedure for visible-light-mediated gold catalyzed C(sp²)-C(sp²)-Coupling (GP4)

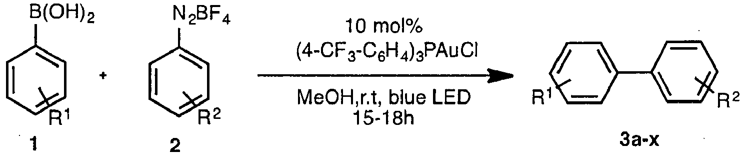

In a dried Pyrex screw-top reaction tube (4-CF₃-C₆H₄)₃PAuCl (0.03 mmol, 10 mol %) and the corresponding boronic acid (0.3 mmol, 1.0 equiv.) were dissolved in 1.5 mL MeOH. After adding the corresponding diazonium salt (1.2 mmol, 4.0 equiv.) the reaction mixture was degassed under argon by sparging for 5-10 min. The tubes were irradiated at room temperature with 29 W blue LEDs for 15-17 hours. The solvent was removed under reduced pressure and the resulting crude product was purified by column chromatography on SiO₂.

### 2.5 General procedure for visible-light-mediated gold catalyzed C(sp²)-C(sp²)-Coupling using BPin as the coupling partner (GP5)

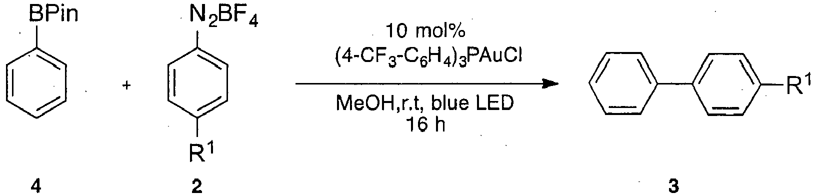

In a dried Pyrex screw-top reaction tube (4-CF₃-C₆H₄)₃PAuCl (0.01 mmol, 10 mol %) and the corresponding boronic pinacol ester (0.1 mmol, 1.0 equiv.) were dissolved in 0.5 mL MeOH. After adding the corresponding diazonium salt (0.4 mmol, 4.0 equiv.) the reaction mixture was degassed under argon by sparging for 5-10 min. The tubes were irradiated at room temperature with 29 W blue LEDs for 16 hours. The solvent was removed under reduced pressure and the resulting crude product was purified by preparative TLC.

### 3. Optimization of model reaction

**Table 2: Screening of photocatalyst.^{[a]}**

| | | | | | | |
|---|---|---|---|---|---|---|
| 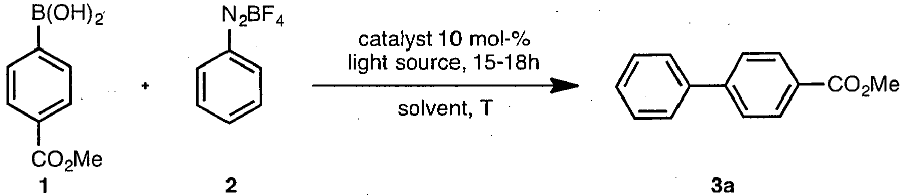 | | | | | | |

| Entry | Catalyst (10 mol-%) | Solvent | T[°C] | Light source | Additives | Yield [%] |
|---|---|---|---|---|---|---|
| 1 | Ph₃PAuCl | MeCN | r.t | Blue LEDs | - | traces^{[d]} |
| 2 | (4-F-C₆H₄)₃PAuCl | MeCN | r.t | Blue LEDs | - | traces^{[d]} |
| 3 | (4-CF₃-C₆H₄)₃PAuCl | MeCN | r.t | Blue LEDs | - | 51^{[c]} |
| 4 | (4-Me-C₆H₄)₃PAuCl | MeCN | r.t | Blue LEDs | - | 20^{[c]} |
| 5 | Ph₂qnPAuCl | MeCN | r.t | Blue LEDs | - | traces^{[d]} |
| 6 | Cy₃PAuCl | MeCN | r.t | Blue LEDs | - | 31^{[c]} |
| 7 | Ph₃PAuNtf₂ | MeCN | r.t | Blue LEDs | - | 31^{[c]} |
| 8 | (4-CF3-C₆H₄)₃PAuNtf₂ | MeCN | r.t | Blue LEDs | - | 22^{[c]} |
| 9 | RO₃PAuCl[b] | MeCN | r.t | Blue LEDs | - | ND^{[d]} |

| | | | | | | |
|---|---|---|---|---|---|---|
| [a] Reaction conditions: 4-methoxycarbonylphenyl boronic acid (1, 0.1 mmol), phenyldiazonium salt (2, 0.4 mmol) and gold catalyst (10 mol %) were reacted in 0.5 mL MeCN at room temperature under irradiation with blue LED. [b] R = 1,3-di-tert-butylbenzene. [c] Yield of isolated product using PTLC. [d] Not detected, determined using GC-MS. | | | | | | |

**Table 3: Screening of solvent.^{[a]}**

| | | | | | | |
|---|---|---|---|---|---|---|
| 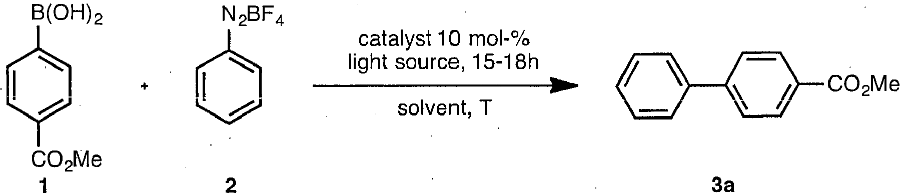 | | | | | | |

| Entry | Catalyst (10 mol-%) | Solvent | T [°C] | Light source | Additives | Yield [%] |
|---|---|---|---|---|---|---|
| 1 | (4-CF₃-C₆H₄)₃PAuCl | MeCN | r.t | Blue LEDs | - | 51^{[b]} |
| 2 | (4-CF₃-C₆H₄)₃PAuCl | DMF | r.t | Blue LEDs | - | ND^{[c]} |
| 3 | (4-CF₃-C₆H₄)₃PAuCl | MeOH | r.t | Blue LEDs | - | 85^{[b]} |
| 4 | (4-CF₃-C₆H₄)₃PAuCl | THF | r.t | Blue LEDs | - | ND^{[c]} |
| 5 | (4-CF₃-C₆H₄)₃PAuCl | DCM | r.t | Blue LEDs | - | ND^{[c]} |
| 6 | (4-CF₃-C₆H₄)₃PAuCl | MeCN | r.t | Blue LEDs | 2 equiv. H2O | 21^{[b]} |

| | | | | | | |
|---|---|---|---|---|---|---|
| [a] Reaction conditions: 4-methoxycarbonylphenyl boronic acid (1, 0.1 mmol), phenyldiazonium salt (2, 0.4 mmol) and gold catalyst (10 mol %) were reacted with different solvents at room temperature under irradiation with blue LED. [b] Yield of isolated product using PTLC. [c] Not detected, determined using GC-MS. | | | | | | |

**Table 4: Screening of different light sources and temperatures.^{[a]}**

| | | | | | | |
|---|---|---|---|---|---|---|
| 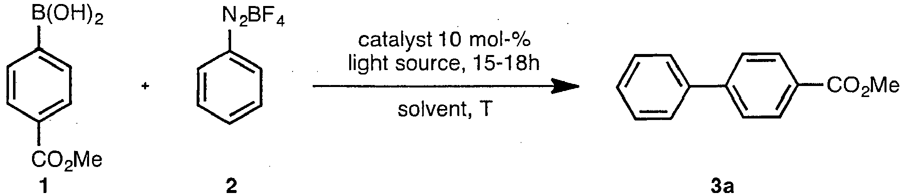 | | | | | | |

| Entry | Catalyst (10 mol-%) | Solvent | T[°C] | Light source | Additives | Yield [%] |
|---|---|---|---|---|---|---|
| 1 | (4-CF₃-C₆H₄)₃PAuCl | MeOH | r.t | Blue LED | - | 85^{[b]} |
| 2 | (4-CF₃-C₆H₄)₃PAuCl | MeOH | r.t | dark | - | ND^{[c]} |
| 3 | (4-CF₃-C₆H₄)₃PAuCl | MeOH | 70°C | CFL | - | ND^{[c]} |
| 4 | (4-CF₃-C₆H₄)₃PAuCl | MeOH | 70°C | dark | - | ND^{[c]} |
| 5 | (4-CF₃-C₆H₄)₃PAuCl | MeOH | 50°C | dark | - | ND^{[c]} |
| 6 | (4-CF₃-C₆H₄)₃PAuCl | MeOH | r.t | UVA | - | 61^{[b]} |
| 7 | (4-CF₃-C₆H₄)₃PAuCl | MeOH | r.t | UV-light[e] | - | 75^{[b]} |

| | | | | | | |
|---|---|---|---|---|---|---|
| [a] Reaction conditions: 4-methoxycarbonylphenyl boronic acid (1, 0.1 mmol), phenyldiazonium salt (2, 0.4 mmol) and gold catalyst (10 mo! %) were reacted in 0.5 mL of MeOH with different light sources temperatures. [b] Yield of isolated product using PTLC. [c] Not detected, determined using GC-MS. [d] λ = 350 nm. [e) λ = 420 nm. | | | | | | |

**Table 5. Variation of equivalents of 2 and gold catalyst (4-CF₃-C₆H₄)₃PAuCl.^{[a][b]}**

| | | | | | | | |
|---|---|---|---|---|---|---|---|
| 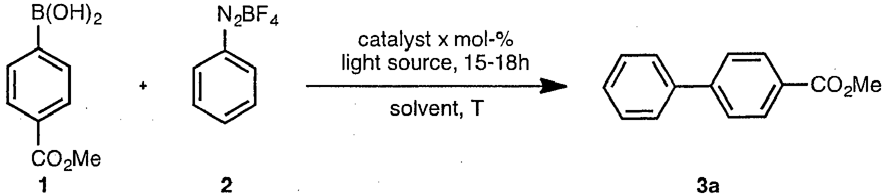 | | | | | | | |

| Entry | Catalyst x mol % | Solvent | T [°C] | Light source | Additives | Diazonium salt x equiv. | Yield [%] |
|---|---|---|---|---|---|---|---|
| 1 | 10 | MeOH | r.t | Blue LEDs | - | 1 | 21 |
| 2 | 10 | MeOH | r.t | Blue LEDs | - | 2 | 54 |
| 3 | 10 | MeOH | r.t | Blue LEDs | - | 3 | 56 |
| 4 | 10 | MeOH | r.t | Blue LEDs | - | 4 | 85 |
| 5 | 5 | MeOH | r.t | Blue LEDs | - | 4 | 47 |
| 6 | - | MeOH | r.t | Blue LEDs | - | 4 | ND^{[c]} |

| | | | | | | | |
|---|---|---|---|---|---|---|---|
| [a] Reaction conditions: 4-methoxycarbonylphenyl boronic acid (1, x mmol), phenyldiazonium salt (2, 0.4 mmol) and gold catalyst (x mol %) were reacted in methanol at room temperature under irradiation with blue LED. [b] Yield of isolated product using PTLC. [c] Not detected, determined using GC-MS. | | | | | | | |

### 4. Synthesis and characterization of cross-coupled substituted biaryls

### 4.1 Synthesis of methyl [1,1'-biphenyl]-4-carboxylate

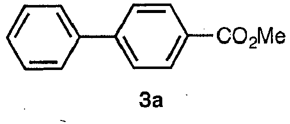

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated at room temperature with blue LEDs for 17 h and the crude product was purified by flash column chromatography (SiO₂, PE/EA, 300:1) to give 53.2 mg of 3a (0.25 mmol, 84 %) as a pale yellow solid. ¹H NMR (300 MHz, CDCl3): δ = 3.90 (s, 3H), 7.33-7.46 (m, 3H), 7.58-7.70 (m, 4H) ppm, 8.05-8.09 (m, 2H).

### 4.2 Synthesis of 4-(trifluoromethyl)-1,1'-biphenyl

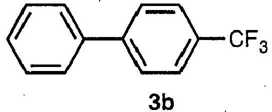

According to GP4, (4-(trifluoromethyl)phenyl)boronic acid (0.3 mmol, 57.0 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 200:1) to give 45.1 mg of 3b (0.20 mmol, 68 %) as a white solid. ¹H NMR (400 MHz, CDCl₃): δ = 7.39-7.43 (m, 1 H), 7.46-7.50 (m, 2H), 7.60-7.62 (m, 2H), 7.70 (s, 4H) ppm.

### 4.3 Synthesis of 1-([1,1'-biphenyl]-4-yl)ethan-1-one

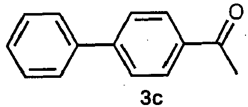

According to GP4, (4-acetylphenyl)boronic acid (0.3 mmol, 49.2 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 500:1) to give 34.3 mg of 3c (0.18 mmol, 59 %) as a white solid. ¹H NMR (300 MHz, CDCl₃): δ = 2.64 (s, 3H), 7.44-7.50 (m, 3H), 7.61-7.71 (m, 4H), 8.01-8.05 (m, 2H) ppm.

### 4.4 Synthesis of [1,1'-biphenyl]-4-carbonitrile

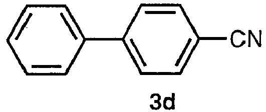

According to GP4, (4-cyanophenyl)boronic acid (0.3 mmol, 44.1 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 16 h and the crude product was purified by flash column chromatography (SiO₂, 100 % PE) to give 31.2 mg of 3d (0.17 mmol, 58 %) as a white solid. ¹H NMR (300 MHz, CDCl₃): δ = 7.41-7.52 (m, 3H), 7.57-7.61 (m, 2H), 7.67-7.75 (m, 4H) ppm.

### 4.5 Synthesis of 4-(methylsulfonyl)-1,1'-biphenyl

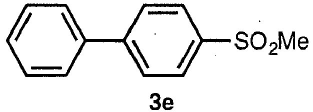

According to GP4, (4-(methylsulfonyl)phenyl)boronic acid (0.3 mmol, 60.0 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 300:1- 10:1) to give 43.2 mg of 3e (0.19 mmol, 62 %) as an off white solid. ¹H NMR (400 MHz, CDCl₃): δ = 3.09 (s, 3H), 7.41-7.51 (m, 3H), 7.60-7.62 (m, 2H), 7.76-7.79 (m, 2H), 7.99-8.04 (m, 2H) ppm.

### 4.6 Synthesis of 3-phenylthiophene-2-carbaldehyde

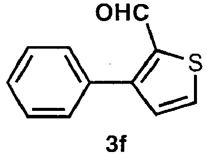

According to GP4, (2-formylthiophen-3-yl)boronic acid (0.3 mmol, 46.8 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 20:1) to give 25.4 mg of 3f (0.14 mmol, 45 %) as a pale yellow solid. ¹H NMR (400 MHz, CDCl₃): δ = 7.38-7.46 (m, 4H), 7.66-7.69 (m, 2H) ppm, 7.74 (d, J = 3.9 Hz, 1 H), 9.90 (s, 1 H) ppm.

### 4.7 Synthesis of 4-fluoro-1,1'-biphenyl

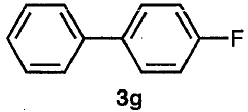

According to GP4, (4-fluorophenyl)boronic acid (0.3 mmol, 42.0 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 15 h and the crude product was purified by flash column chromatography (SiO₂, 100 % PE) to give 24.8 mg of 3g (0.15 mmol, 48 %) as a white solid. ¹H NMR (300 MHz, CDCl₃): δ = 7.10-7.16 (m, 2H), 7.31-7.37 (m, 1 H) ppm, 7.41-7.47 (m, 2H), 7.52-7.59 (m, 4H) ppm.

### 4.8 Synthesis of 4-chloro-1,1'-biphenyl

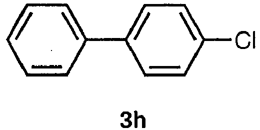

According to GP4, (4-chlorophenyl)boronic acid (0.3 mmol, 47.0 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 500:1) to give 49.7 mg of 3h (0.26 mmol, 88 %) as a white solid. ¹H NMR (300 MHz, CDCl3): δ = 7.33-7.47 (m, 5H), 7.50-7.62 (m, 4H) ppm.

### 4.9 Synthesis of 4-bromo-1,1'-biphenyl

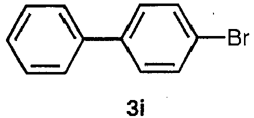

According to GP4, (4-bromophenyl)boronic acid (0.3 mmol, 60.2 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, f4.0 equiv.) the reaction mixture was degassed under argon by sparging for 5-10 min. The tubes were irradiated room temperature with blue LEDs for 18 h and the crude product was purified by flash column chromatography (SiO₂, 100 % PE) to give 55.8 mg of 3i (0.26 mmol, 80 %) as an off white solid. ¹H NMR (300 MHz, CDCl3): δ = 7.34-7.48 (m, 5H), 7.54-7.58 (m, 4H) ppm.

### 4.10 Synthesis of 4-methoxy-1,1'-biphenyl

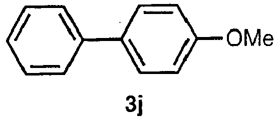

According to GP4, (4-methoxyphenyl)boronic acid (0.3 mmol, 45.6 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 17 h and the crude product was purified by flash column chromatography (SiO₂, PE/EA, 150:1) to give 12.1 mg of 3j (0.07 mmol, 23 %) as a yellow solid. ¹H NMR (300 MHz, CDCl₃): δ = 3.86 (s, 3H), 6.96-7.01 (m, 2H), 7.27-7.33 (m, 1 H), 7.39-7.44 (m, 2H), 7.51-7.57 (m, 4H) ppm.

### 4.11 Synthesis of methyl [1,1'-biphenyl]-3-carboxylate

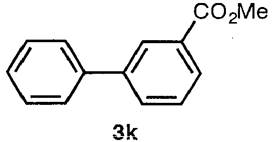

According to GP4, (3-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 15 h and the crude product was purified by flash column chromatography (SiO₂, PE/EA, 150:1) to give 26.3 mg of 3k (0.12 mmol, 41 %) as a colorless oil. ¹H NMR (300 MHz, CDCl₃): δ = 3.95 (s, 3H), 7.36-7.41 (m, 1H), 7.44-7.54 (m, 3H), 7.61-7.65 (m, 2H), 7.77-7.81 (m, 1H), 8.01-8.05 (m, 1H), 8.29 (t, J = 1.7 Hz, 1 H) ppm.

### 4.12 Synthesis of methyl [1,1'-biphenyl]-2-carboxylate

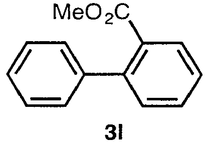

According to GP4, (2-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding benzenediazonium tetrafluoroborate (1.2 mmol, 230 mg, 4.0 equiv.) the reaction mixture was degassed under argon by sparging for 5-10 min. The tubes were irradiated room temperature with blue LEDs for 15 h and the crude product was purified by flash column chromatography (SiO₂, PE/EA, 200:1) to give 42.8 mg of 3I (0.20 mmol, 67 %) as a pale yellow oil. ¹H NMR (300 MHz, CDCl₃): δ = 3.65 (s, 3H), 7.31-7.45 (m, 7H), 7.44-7.54 (m, 3H), 7.61-7.65 (m, 2H), 7.54 (td, J = 1.4 Hz, 7.6 Hz, 1 H), 7.84 (dd, J = 1.2 Hz, 7.6 Hz, 1 H) ppm.

### 4.13 Synthesis of methyl 4'-(trifluoromethyl)-[1,1'-biphenyl]-4-carboxylate

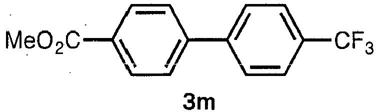

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-(trifluoromethyl)benzenediazonium tetrafluoroborate (1.2 mmol, 312 mg, 4.0 equiv.) the reaction mixture was degassed under argon by sparging for 5-10 min. The tubes were irradiated room temperature with blue LEDs for 15 h and the crude product was purified by flash column chromatography (SiO₂, PE/EA, 250:1) to give 68.8 mg of 3m (0.25 mmol, 82 %) as a white solid. M.p = 121-122 °C. ¹H NMR (400 MHz, CDCl₃): δ = 3.95 (s, 3H), 7.65-7.68 (m, 2H), 7.72 (s, 4H), 8.12-8.15 (m, 2H) ppm. ¹³C NMR (101 MHz, CDCl₃): δ = 52.2 (q), 125.9 (s, q: JC-F = 3.8 Hz), 127.2 (d), 127.6 (d), 129.9 (s), 130.3 (d), 143.6 (s), 144.1 (s), 166.7 (s) ppm. ¹⁹F NMR (283 MHz, CDCl₃): δ = -62.5 (s, 3F) ppm. IR (ATR): ṽ = 2954, 1943, 1712, 1609, 1584, 1437, 1398, 1373, 1334, 1287, 1182, 1158, 1143, 1111, 1075, 1023, 1008, 956, 869, 842, 833, 774, 739, 700, 667 cm-1. HR MS (EI (+)): m/z = 280.0695, calcd. for [C₁₅H₁₁O₂F₃]⁺: 280.0706.

### 4.14 Synthesis of methyl 4'-fluoro-[1,1'-biphenyl]-4-carboxylate

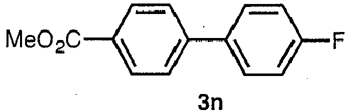

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-fluorobenzenediazonium tetrafluoroborate (1.2 mmol, 252 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 200:1) to give 52.9 mg of 3n (0.23 mmol, 77 %) as a white solid. ¹H NMR (600 MHz, CDCl₃): δ = 3.94 (s, 3H), 7.15 (t, J = 8.6 Hz, 2H), 7.57-7.62 (m, 4H), 8.10 (d, J = 8.2 Hz, 2H) ppm.

### 4.15 Synthesis of methyl 4'-bromo-[1,1'-biphenyl]-4-carboxylate

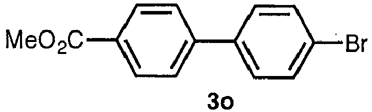

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-bromobenzenediazonium tetrafluoroborate (1.2 mmol, 325 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 100:1) to give 83.1 mg of 3o (0.29 mmol, 95 %) as a white solid. ¹H NMR (300 MHz, CDCl₃): δ = 3.95 (s, 3H), 7.47-7.50 (m, 2H), 7.57-7.64 (m, 4H), 8.09-8.12 (m, 2H) ppm.

### 4.16 Synthesis of methyl 4'-chloro-[1,1'-biphenyl]-4-carboxylate

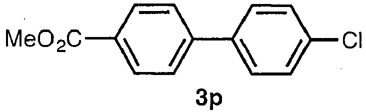

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-chlorobenzenediazonium tetrafluoroborate (1.2 mmol, 272 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 200:1) to give 63.8 mg of 3p (0.26 mmol, 84 %) as an off white solid. ¹H NMR (300 MHz, CDCl₃): δ = 3.95 (s, 3H), 7.40-7.45 (m, 2H), 7.53-7.63 (m, 4H), 8.09-8.13 (m, 2H) ppm.

### 4.17 Synthesis of methyl 4'-(tert-butyl)-[1,1'-biphenyl]-4-carboxylate

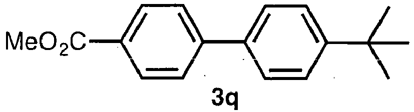

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-(tert-butyl)benzenediazonium tetrafluoroborate (1.2 mmol, 298 mg, 4.0 equiv.) the reaction mixture was degassed under argon by sparging for 5-10 min. The tubes were irradiated room temperature with blue LEDs for 15 h and the crude product was purified by flash column chromatography (SiO₂, PE/EA, 100:1) to give 61.8 mg of 3q (0.23 mmol, 77 %) as an off white solid. ¹H NMR (300 MHz, CDCl₃): δ = 1.37 (s, 9H), 3.94 (s, 3H), 7.48-7.50 (m, 2H), 7.57-7.59 (m, 2H), 7.65-7.67 (m, 2H), 8.07-8.11 (m, 2H) ppm.

### 4.18 Synthesis of methyl 4'-methoxy-[1,1'-biphenyl]-4-carboxylate

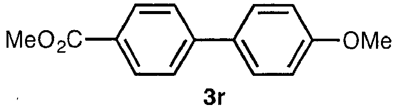

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-methoxybenzenediazonium tetrafluoroborate (1.2 mmol, 266 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 50:1) to give 21.7 mg of 3r (0.09 mmol, 30 %) as a pale yellow solid. ¹H NMR (400 MHz, CDCl₃): δ = 3.87 (s, 3H), 3.94 (s, 3H), 6.98-7.02 (m, 2H), 7.56-7.64 (m, 4H), 8.07-8.11 (m, 2H) ppm.

### 4.19 Synthesis of methyl 4'-(methylsulfonyl)-[1,1'-biphenyl]-4-carboxylate

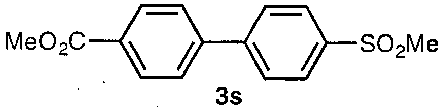

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-(methylsulfonyl)benzenediazonium tetrafluoroborate (1.2 mmol, 324 mg, 4.0 equiv.) the reaction mixture was degassed under argon by sparging for 5-10 min. The tubes were irradiated room temperature with blue LEDs for 17 h and the crude product was purified by flash column chromatography (SiO₂, 100% DCM) to give 50.6 mg of 3s (0.17 mmol, 58 %) as an off white solid. M.p = 196-197 °C. ¹H NMR (300 MHz, CDCl₃): δ = 3.10 (s, 3H), 3.96 (s, 3H), 7.68 (d, J = 8.8 Hz, 2H), 7.80 (d, J = 8.8 Hz, 2H), 8.04 (d, J = 8.8 Hz, 2H), 8.16 (d, J = 8.3 Hz, 2H) ppm. ¹³C NMR (75 MHz, CDCl₃): δ = 44.5 (q), 52.2 (q), 127.3 (d), 128.0 (d), 128.1 (d), 130.2 (s), 130.3 (d), 139.9 (s), 143.3 (s), 145.4 (s), 166.5 (s) ppm. IR (ATR): ṽ = 3073, 3019, 2961, 2933 1946, 1925,1715,1608,1580,1561,1456,1440,1396,1311,1294,1273,1214,1196,1181, 1150, 1117, 1096, 1021, 1005, 970, 867, 833, 784, 869, 751, 714, 699, 615 cm.1. HR MS (EI (+)): m/z = 290.0599, calcd. for [C₁₅H₁₄O₄S]⁺: 290.0607.

### 4.20 Synthesis of methyl 3-(4-(methoxycarbonyl)phenyl)thiophene-2-carboxylate

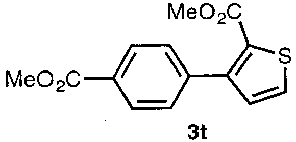

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 2-(methoxycarbonyl)-3-thiophenediazonium tetrafluoroborate (1.2 mmol, 306 mg, 4.0 equiv.) the reaction mixture was degassed under argon by sparging for 5-10 min. The tubes were irradiated room temperature with blue LEDs for 15 h and the crude product was purified by flash column chromatography (SiO₂, PE/EA, 150:1 till 50:1) to give 40.6 mg of 3t (0.15 mmol, 49 %) as a white solid. M.p = 127-128 °C. ¹H NMR (400 MHz, CDCl3): δ = 3.77 (s, 3H), 3.94 (s, 3H), 7.10 (d, J = 5.0 Hz, 1 H), 7.50-7.55 (m, 3H), 8.06-8.09 (m, 2H) ppm. ¹³C NMR (101 MHz, CDCl₃): δ = 52.0 (q), 52.1 (q), 127.8 (d), 129.1 (d), 129.3 (d), 129.5 (s), 130.5 (d), 131.2 (d), 133.3 (s), 140.4 (s), 147.3 (s), 162.2 (s), 166.9 (s) ppm. IR (ATR): ṽ = 3107, 3026, 2954, 2841, 1712, 1610, 1570, 1540, 1498, 1458, 1430, 1416, 1403, 1317, 1271, 1224, 1181, 1099, 1068, 1018, 966, 893, 865, 843, 819, 786, 763, 710, 700, 676,654, 628 cm-1. HR MS (EI (+)): m/z = 276.0437, calcd. for [C₁₄H₁₂O₄S]⁺: 276.0450.

### 4.21 Synthesis of methyl 4'-acetyl-[1,1'-biphenyl]-4-carboxylate

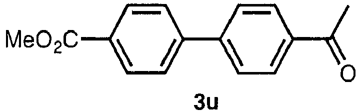

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-acetylbenzenediazonium tetrafluoroborate (1.2 mmol, 281 mg, 4.0 equiv.) the reaction mixture was degassed under argon by sparging for 5-10 min. The tubes were irradiated room temperature with blue LEDs for 17 h and the crude product was purified by flash column chromatography (SiO₂, PE/DCM, 10:1) to give 56.7 mg of 3u (0.22 mmol, 75 %) as a white solid. ¹H NMR (300 MHz, CDCl₃): δ = 2.65 (s, 3H), 3.95 (s, 3H), 7.68-7.74 (m, 4H), 8.04-8.08 (m, 2H), 8.12-8-16 (m, 2H) ppm.

### 4.22 Synthesis of methyl 3'-fluoro-[1,1'-biphenyl]-4-carboxylate

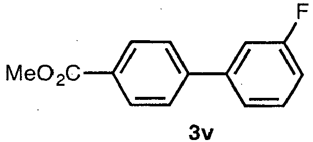

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 3-fluorobenzenediazonium tetrafluoroborate (1.2 mmol, 252 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 150:1) to give 57.0 mg of 3v (0.25 mmol, 83 %) as an off white solid. M.p = 59-60 °C. ¹H NMR (600 MHz, CDCl₃): δ = 3.94 (s, 3H), 7.07-7.10 (m, 1 H), 7.32 (dt, J = 1.9 Hz, J = 9.9 Hz, 1 H), 7.39-7.45 (m, 2H), 7.63-7.65 (m, 2H), 8.10-8.12 (m, 2H) ppm. ¹³C NMR (151 MHz, CDCl₃): δ = 52.5 (q), 114.5 (d, d: JC-F = 23.4 Hz), 115.3 (d, d: JC-F = 21.0 Hz), 123.2 (d, d: JC-F = 3.0 Hz), 127.4 (d), 129.8 (s), 130.5 (d), 130.7 (d, d: JC-F = 8.5 Hz), 142.5 (s, d: JC-F = 7.4 Hz), 144.5 (s, d: JC-F = 2.3 Hz), 163.5 (s, d: JC-F = 245.3 Hz), 167.2 (s) ppm. ¹⁹F NMR (283 MHz, CDCl₃): δ = -112.7 (s, 1 F) ppm. IR (ATR): ṽ = 3075, 3008, 2957, 2852, 1937, 1719, 1611, 1589, 1569, 1486, 1475, 1439, 1399, 1279, 1189, 1166, 1114, 1037, 1016, 1000, 961, 903, 881, 854, 828, 797, 770, 726, 700, 685, 648 cm-1. HR MS (EI (+)): m/z = 230.0740, calcd. for [C₁₄H₁₁O₂F]⁺: 230.0743.

### 4.23 Synthesis of methyl 2'-fluoro-[1,1'-biphenyl]-4-carboxylate

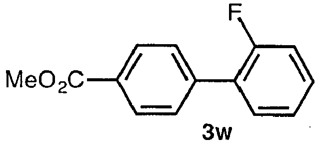

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 2-fluorobenzenediazonium tetrafluoroborate (1.2 mmol, 252 mg, 4.0 equiv.) the reaction mixture was degassed under argon by sparging for 5-10 min. The tubes were irradiated room temperature with blue LEDs for 15 h and the crude product was purified by flash column chromatography (SiO₂, PE/EA, 150:1) to give 45.3 mg of 3w (0.20 mmol, 66 %) as a pale brown solid. M.p = 61-62 °C. ¹H NMR (600 MHz, CDCl₃): δ = 3.94 (s, 3H), 7.16-7.19 (m, 1H), 7.23 (td, J = 1.0 Hz, J = 7.5 Hz, 1H), 7.33-7.38 (m, 1 H), 7.46 (td, J = 1.7 Hz, J = 7.7 Hz, 1 H), 7.63 (dd, J = 1.5 Hz, J = 8.3 Hz, 2H), 8.10 (d, J = 8.4 Hz, 2H) ppm. ¹³C NMR (151 MHz, CDCl₃): δ = 52.5 (q), 116.5 (d, d: JC-F = 23.8 Hz), 124.8 (d, d: JC-F = 3.7 Hz), 128.3 (d, d: JC-F = 12.8 Hz), 129.3 (d, d: JC-F = 2.8 Hz), 129.5 (s), 130.0 (d), 130.1 (d, d: JC-F = 8.3 Hz), 130.9 (s, d: JC-F = 3.2 Hz), 140.7 (s), 160.0 (s, d: JC-F = 247.2 Hz), 167.2 (s) ppm. ¹⁹F NMR (283 MHz, CDCl₃): δ = -117.5 (s, 1F) ppm. IR (ATR): ṽ = 3002, 2954, 2851, 1939, 1720, 1613, 1584, 1514, 1485, 1453, 1440, 1402, 1316, 1282, 1253, 1209, 1116,1102, 1043, 1025, 1008, 972, 949, 873, 857, 832, 818, 777, 766, 756, 726, 703, 616 cm-1. H R MS (EI (+)): m/z = 230.0722, calcd. for [C₁₄H₁₁O₂F]⁺: 230.0738.

### 4.24 Synthesis of methyl 4'-methyl-[1,1'-biphenyl]-4-carboxylate

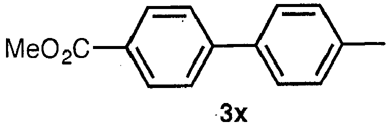

According to GP4, (4-(methoxycarbonyl)phenyl)boronic acid (0.3 mmol, 54.0 mg, 1.0 equiv.) and (4-CF₃-C₆H₄)₃PAuCl (0.03 mmol, 21.0 mg, 10 mol %) were dissolved in 1.5 mL MeOH. After adding 4-methylbenzenediazonium tetrafluoroborate (1.2 mmol, 247 mg, 4.0 equiv.) the reaction mixture was degassed under argon by sparging for 5-10 min. The tubes were irradiated room temperature with blue LEDs for 16 h and the crude product was purified by flash column chromatography (SiO₂, PE/EA, 200:1) to give 24.8 mg of 3x (0.11 mmol, 38 %) as a pale yellow solid. ¹H NMR (300 MHz, CDCl₃): δ = 2.41 (s, 3H), 3.94 (s, 3H), 7.29 (s, 2H), 7.51-7.54 (d, J = 8.2 Hz, 2H), 7.63-7.66 (m, 2H), 8.07-8.10 (m, 2H) ppm.

### 4.25 Synthesis of 4-iodo-1,1'-biphenyl

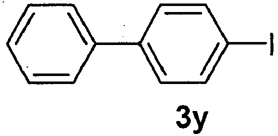

The reaction was carried out according to GP4, using 74.3 mg of (4-iodophenyl)boronic acid (0.3 mmol, 1.0 equiv.), 21.0 mg of (4-CF₃-C₆H₄)₃PAuCl (0.03 mmol, 10 mol %), 230 mg of benzenediazonium tetrafluoroborate (1.2 mmol, 4.0 euqiv.) and 1.5 mL of MeOH. After flash column chromatography (SiO₂, 100 % *n*-heptane), 68.0 mg of 3y (0.24 mmol, 81 %) were isolated as a white solid. ¹H NMR (300 MHz, CDCl₃): δ = 7.33-7.39 (m, 3H), 7.44-7.47 (m, 2H), 7.55-7.57 (m, 1 H), 7.60-7.62 (m, 1 H), 7.76-7.79 (m, 2H) ppm. The data is consistent with literature values.

### 5. Mechanistic studies

### 5.1 Control experiments

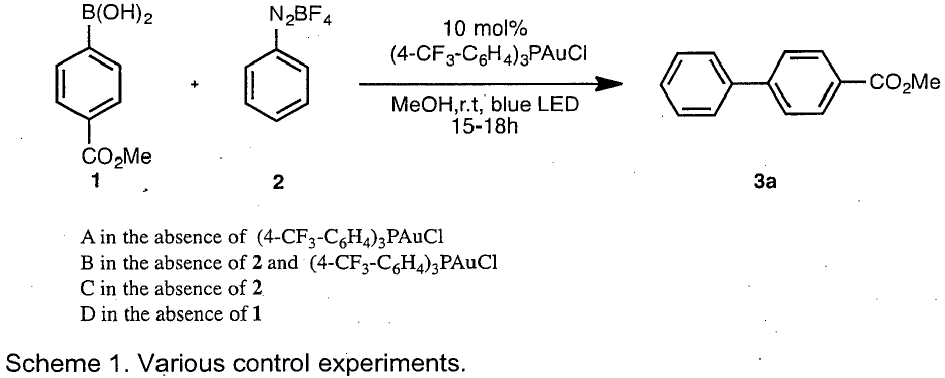

### Control experiment A:

In a dried Pyrex screw-top reaction tube (4-(methoxycarbonyl)phenyl)boronic acid (0.1 mmol, 1.0 equiv.) was dissolved in 0.5 mL MeOH. After addition of benzenediazonium tetrafluoroborate (0.4 mmol, 0.4 equiv.) the reaction mixture was degassed under argon by sparging for 5-10min. The tube was irradiated with 29W blue LEDs for 16h. The crude mixture was subjected to GC-MS analysis, no product 3a was detected. This observation was also confirmed by NMR spectroscopy, which shows that the presence of the catalyst is essential to the reaction.

### Control experiment B:

In a dried Pyrex screw-top reaction tube (4-(methoxycarbonyl)phenyl)boronic acid (0.1 mmol, 1.0 equiv.) was dissolved in 0.5 mL MeOH. After the reaction mixture was degassed under argon by sparging for 5-10min, the tube was irradiated with 29W blue LEDs for 16h. The crude mixture was analyzed by GC-MS and NMR spectroscopy, the intact boronic acid and the corresponding hydrogenated product, methyl benzoate, could be detected.

### Control experiment C:

In a dried Pyrex screw-top reaction tube (4-CF₃-C₆H₄)₃PAuCl (0.01 mmol, 10 mol %) and (4-(methoxycarbonyl)phenyl)boronic acid (0.1 mmol, 1.0 equiv.) were dissolved in 0.5 mL MeOH. After the reaction mixture was degassed under argon by sparging for 5-10min, the tube was irradiated with 29W blue LEDs for 15h. The solvent was removed under reduced pressure and the crude product was analyzed by ¹H NMR, ¹¹B NMR, ³¹P NMR and ¹⁹F NMR, which indicate an intact catalyst and boronic acid. This shows that the presence of the aryldiazonium salt is essential to the reaction.

### Control experiment D:

In a dried Pyrex screw-top reaction tube (4-CF₃-C₆H₄)₃PAuCl (0.01 mmol, 10 mol %) was dissolved in 0.5 mL MeOH. After addition of benzenediazonium tetrafluoroborate (0.4 mmol, 0.4 equiv.) the reaction mixture was degassed under argon by sparging for 5-10min. The tube was irradiated with 29W blue LEDs for 16h. The crude mixture was subjected to GC-MS analysis which showed that homocoupling product was obtained.

### 5.2 Variation of the counterion of the aryldiazonium salt

To answer the question whether the tetrafluoroborate anion plays an essential role in the reaction mechanism, a diazonium salt with bis((trifluoromethyl)sulfonyl)amide as the anion was synthesized. The synthesis was performed according to a procedure reported by Hass et al. (A. Haas, Y. L. Yagupolskii, C. Klare, Mendeleev Commun. 1992, 2, 70.).

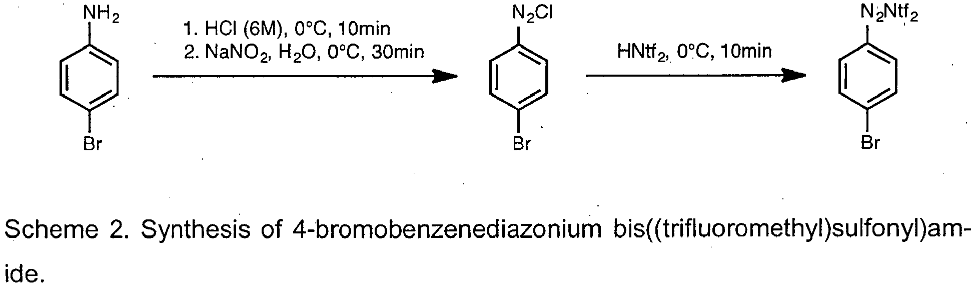

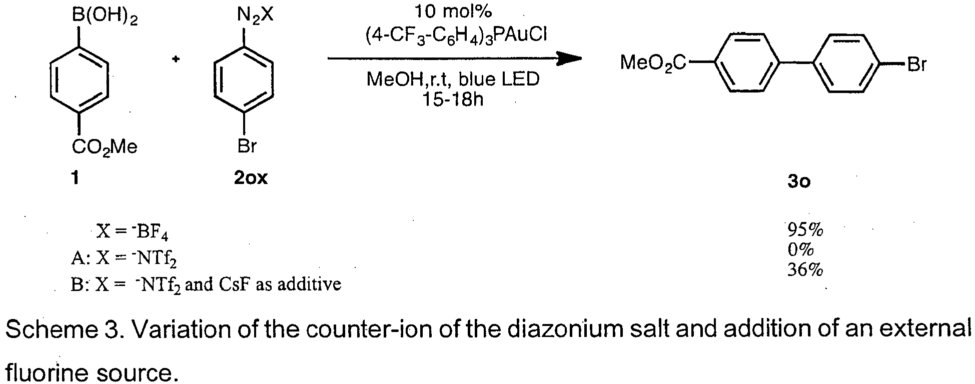

### Experiment A:

In a dried Pyrex screw-top reaction tube (4-CF₃-C₆H₄)₃PAuCl (0.01 mmol, 10 mol %) and (4-(methoxycarbonyl)phenyl)boronic acid (0.1 mmol, 1.0 equiv.) were dissolved in 0.5 mL MeOH. After adding 4-bromobenzenediazonium bis((trifluoromethyl)sulfonyl)amide (0.4 mmol, 0.4 equiv.) the reaction mixture was degassed under argon by sparging for 5-10 min. The tube was irradiated with 29 W blue LEDs for 15 hours. The crude mixture was subjected to GC-MS analysis, no product 3o was detected. This shows that the presence of a fluoride source, such as tetrafluoroborate, is essential for the reaction.

### Experiment B:

In a dried Pyrex screw-top reaction tube (4-CF₃-C₆H₄)₃PAuCl (0.01 mmol, 10 mol %) and (4-(methoxycarbonyl)phenyl)boronic acid (0.1 mmol, 1.0 equiv.) were dissolved in 0.5 mL MeOH. After adding CsF (0.2 mmol, 2.0 equiv.) 4-bromobenzenediazonium bis((trifluoromethyl)sulfonyl)amide (0.4 mmol, 0.4 equiv.) the reaction mixture was degassed under argon by sparging for 5-10 min. The tube was irradiated with 29 W blue LEDs for 15 hours. The crude mixture was subjected to GC-MS analysis, product 3o was detected. The product was purified by pTLC (SiO₂, PE/EA, 5:1) to give 10.5 mg of 3o (0.04 mmol, 36%). This shows, that adding an external fluoride source the reactions proceeds and the desired product can be formed.

### 5.3 Quantum yield measurement

The quantum yield (Φ) was determined by the known ferrioxolate actinometry method. A ferrioxolate actinometry solution was prepared by following the Hammond variation of the Hatchard and Parker procedure outlined in the Handbook of Photochemistry.[18] The irradiated light intensity was estimated to 3.00 x 10⁻⁷ Einstein S⁻¹ by using K₃[Fe(C₂O₄)₃] as an actinometer.

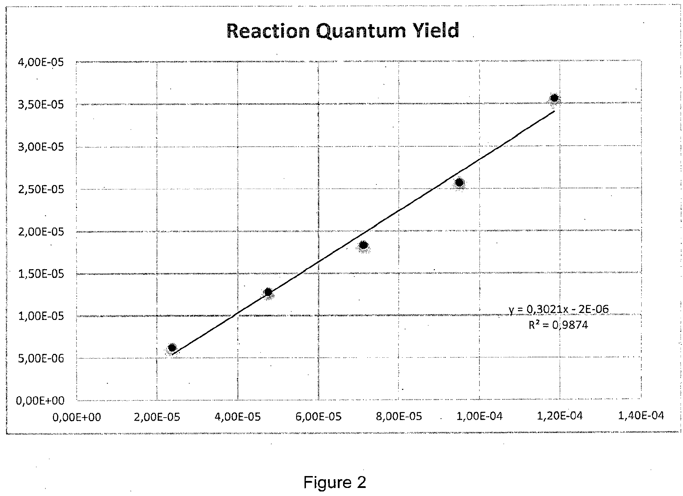

Five dried Pyrex screw-top reaction tubes were each charged with (4-CF₃-C₆H₄)₃PAuCl (8.0 µmol, 10 mol %), (4-(methoxycarbonyl)phenyl)boronic acid (0.08 mmol, 1.0 equiv.) and dodecane (0.08 mmol, 1.0 equiv.) and dissolved in 0.4 mL MeOH. After adding phenyldiazonium tetrafluoroborate (0.32 mmol, 0.4 equiv.) the reaction mixture was degassed under argon by sparging for 5-10 min. The solutions were irradiation with blue LEDs for specified time intervals (5 min, 10 min, 15 min, 20 min and 25 min). The moles of products formed were determined by GC-MS with dodecane as reference standard. The number of moles of products (y axis) per unit time is related to the number of photons (x axis, calculated from the light intensity). The slope of the graph represented in Figure 2 equals the quantum yield (Φ) of the photoreaction. Φ = 0.3021 (=30.2%).

## Claims

1. A method for manufacturing biaryl compounds, comprising the steps:
(a) providing a mixture containing a boron-containing aryl compound represented by the following Formula (i) or a silicon-containing aryl compound represented by the following Formula (ii), an aryldiazonium compound represented by the following Formula (iii) and a gold(I) catalyst in a solvent 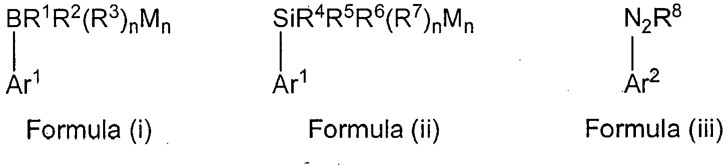 wherein
Ar¹ and Ar² are each independently selected from a C₃-C₁₂ aryl group and a C₃-C₁₂ heteroaryl group, and each group Ar¹ and Ar² may independently contain one or more substituent(s),
in Formula (i) R¹, R² and R³ are each independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, n represents an integer of 0 or 1, wherein two or more of R¹, R² and R³ may be bound to each other to form one or more rings and M represents a cation selected from Li, Na, K and ammonium,
in Formula (ii) R⁴, R⁵, R⁶ and R⁷ are each independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, n represents an integer of 0, 1 or 2, wherein two or more of R⁴, R⁵, R⁶ and R⁷ may be bound to each other to form one or more rings and M represents a cation selected from Li, Na, K and ammonium,
in Formula (iii) R⁸ represents a fluorine-containing counter-ion, and
(b) irradiating the resulting mixture with visible light.

2. The method according to claim 1, wherein the boron-containing compound of Formula (i) is selected from a compound represented by the following Formulae (i-1) to (i-4): 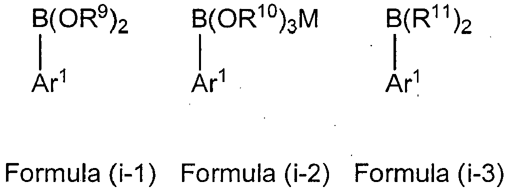 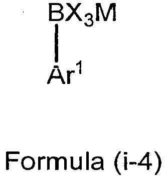 wherein
Ar¹ is as defined above,
in Formula (i-1) each R⁹ is independently selected from hydrogen, C₁-C₁₂ alkyl, C₂-C₁₂ alkenyl, C₂-C₁₂ alkynyl and C₃-C₁₂ aryl, and wherein both R⁹ may be bound to each other to form a ring,
in Formula (i-2) each R¹⁰ is independently selected from H, C₁-C₁₂ alkyl, C₂-C₁₂ alkenyl, C₂-C₁₂ alkynyl and C₃-C₁₂ aryl, wherein two or all of R¹⁰ may be bound to each other to form one or more rings and M represents a cation selected from Li, Na, K and ammonium,
in Formula (i-3) each R¹¹ is independently selected from C₁-C₁₂ alkyl, C₂-C₁₂ alkenyl, C₂-C₁₂ alkynyl and C₃-C₁₂ aryl, and wherein both R¹¹ may be bound to each other to form a ring, and
in Formula (i-4) each X is independently selected from halogen and M represents a cation selected from Li, Na, K and ammonium.

3. The method according to claim 1 or 2, wherein the boron-containing compound of Formula (i) is selected from a compound represented by the following Formulae (i-1-1) to (i-4-1): 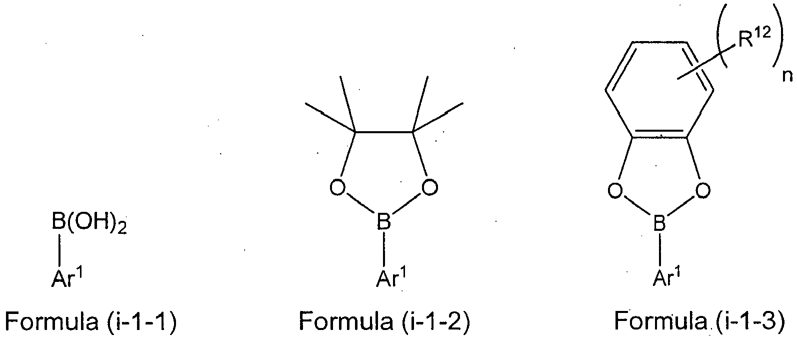 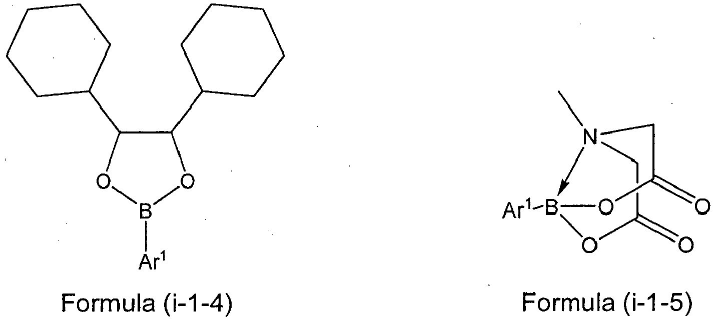 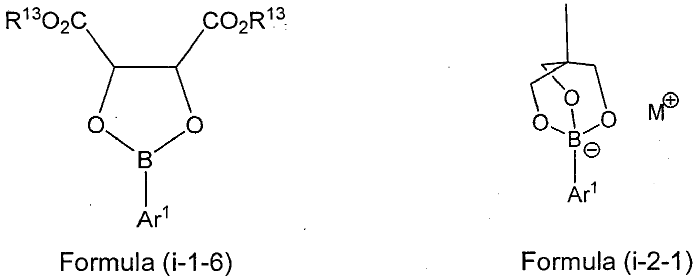 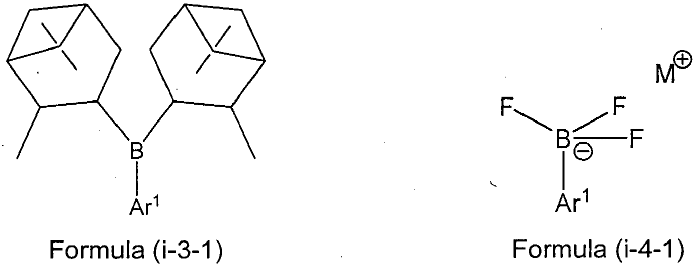 wherein
Ar¹ is as defined above,
in Formula (i-1-3) each R¹² is independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, wherein n represents an integer of 0 to 4 and one or more of R¹² may be bound to each other to form one or more rings,
in Formula (i-1-6) each R¹³ is independently selected from hydrogen, C₁-C₁₂ alkyl, C₂-C₁₂ alkenyl, C₂-C₁₂ alkynyl, and C₃-C₁₂ aryl, and wherein both R¹³ may be bound to each other to form a ring, and
in Formulae (i-2-1) and (i-4-1) M represents a cation selected from Li, Na, K and ammonium.

4. The method according to any one of claims 1 to 4, wherein the silicon-containing compound of Formula (ii) is selected from a compound represented by the following Formula (ii-1) to (ii-4): 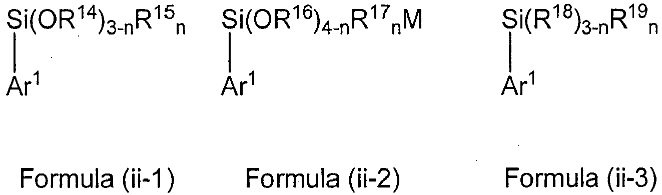 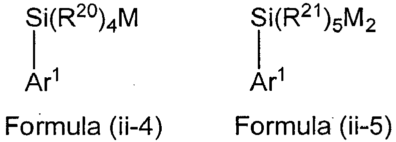 wherein
Ar¹ is as defined above,
in Formula (ii-1) each R¹⁴ is independently selected from H, C₁-C₁₂ alkyl, C₂-C₁₂ alkenyl, C₂-C₁₂ alkynyl and C₃-C₁₂ aryl, each R¹⁵ is independently selected from H, hydroxy, halogen, amino, C₁-C₁₂ alkyl, C₁-C₁₂ alkoxy, C₂-C₁₂ alkenyl, C₂-C₁₂ alkenoxy, C₂-C₁₂ alkynyl, C₂-C₁₂ alkynoxy, C₃-C₁₂ aryl and C₃-C₁₂ aryloxy, wherein two or more of R¹⁴ and R¹⁵ may be bound to each other to form one or more rings and n represents an integer of 0 to 3,
in Formula (ii-2) each R¹⁶ is independently selected from H, C₁-C₁₂ alkyl, C₂-C₁₂ alkenyl, C₂-C₁₂ alkynyl and C₃-C₁₂ aryl, each R¹⁷ is independently selected from H, hydroxy, halogen, amino, C₁-C₁₂ alkyl, C₁-C₁₂ alkoxy, C₂-C₁₂ alkenyl, C₂-C₁₂ alkenoxy, C₂-C₁₂ alkynyl, C₂-C₁₂ alkynoxy, C₃-C₁₂ aryl and C₃-C₁₂ aryloxy, wherein two or more of R¹⁶ and R¹⁷ may be bound to each other to form one or more rings and n represents an integer of 0 to 4,
in Formula (ii-3) each R¹⁸ and R¹⁹ is independently selected from H, hydroxy, halogen, amino, C₁-C₁₂ alkyl, C₂-C₁₂ alkenyl, C₂-C₁₂ alkynyl and C₃-C₁₂ aryl, and wherein two or more of R¹⁸ and R¹⁹ may be bound to each other to form one or more rings,
in Formula (ii-4) each R²⁰ is independently selected from H, hydroxy, halogen, amino, C₁-C₁₂ alkyl, C₂-C₁₂ alkenyl, C₂-C₁₂ alkynyl and C₃-C₁₂ aryl, wherein two or more of R²⁰ may be bound to each other to form one or more rings and M represents a cation selected from Li, Na, K and ammonium, and
in Formula (ii-5) each R²¹ is independently selected from H, hydroxy, halogen, amino, C₁-C₁₂ alkyl, C₂-C₁₂ alkenyl, C₂-C₁₂ alkynyl and C₃-C₁₂ aryl, wherein two or more of R²¹ may be bound to each other to form one or more rings and each M independently represents a cation selected from Li, Na, K and ammonium.

5. The method according to any one of claims 1 to 4, wherein the silicon-containing compound of Formula (ii) is selected from a compound represented by the following Formulae (ii-1-1) to (ii-5-1): 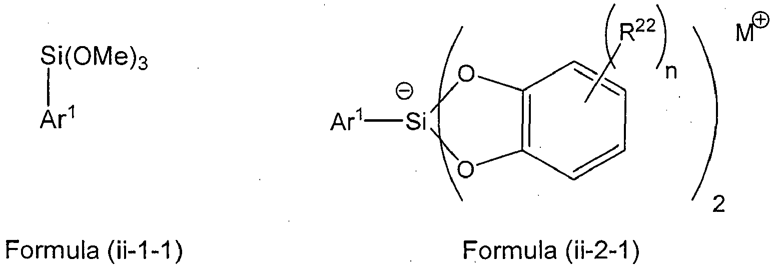 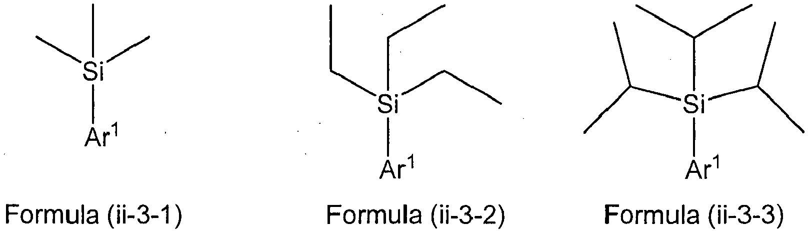 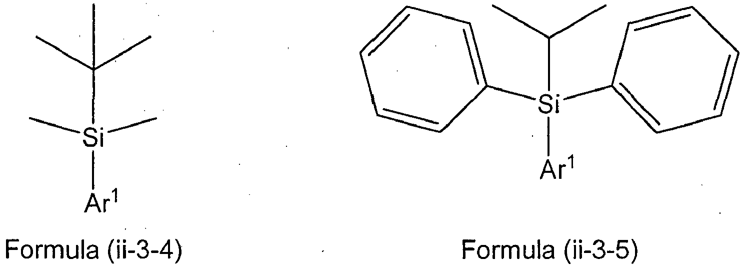 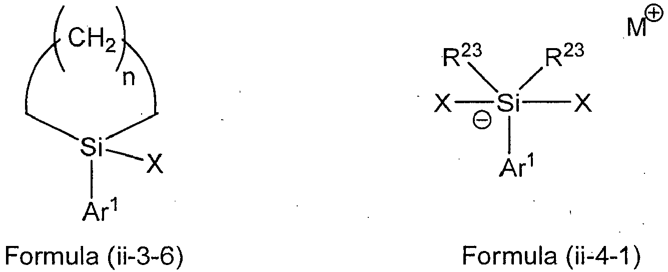 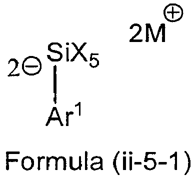 wherein
Ar¹ is as defined above,
in Formula (ii-2-1) each R²² is independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, wherein n represents an integer of 0 to 4, one or more of R²² may be bound to each other to form one or more rings and M represents a cation selected from Li, Na, K and ammonium, in Formula (ii-3-6) X represents halogen and n represents an integer of 1 to 4, in Formula (ii-4-1) each R²³ is independently selected from hydroxy, amino, halogen, C₁-C₁₂ alkyl, C₁-C₁₂ alkoxy, C₂-C₁₂ alkenyl, C₂-C₁₂ alkenyloxy, C₂-C₁₂ alkynyl, C₂-C₁₂ alkynyloxy, C₃-C₁₂ aryl and C₃-C₁₂ aryloxy, X represents halogen, wherein one or more of R²³ may be bound to each other to form one or more rings and M represents a cation selected from Li, Na, K and ammonium, and
in Formula (ii-5-1) X represents halogen and each M represents a cation selected from Li, Na, K and ammonium.

6. The method according to any one of claims 1 to 5, wherein R⁸ is selected from BF₄, PF₆, SbF₆, OTf, NTf₂, OSO₂C₄F₉, F, OSO₂F, BArF20, BArF24, brosylate, carborane, C(TF)₃, B(Ph)₄, Altebat, Bortebat, PFTB, and C(CF₃)₄.

7. The method according to any one of claims 1 to 6, wherein the aryl groups Ar¹ and Ar² are independently selected from furanyl, pyrrolyl, thiophenyl, imidazolyl, pyrazolyl, oxazolyl, isoxazolyl, thiazolyl, phenyl, pyridinyl, pyrazinyl, pyrimidinyl, pyradizinyl, benzofuranyl, indolyl, benzothiophenyl, benzimidazolyl, indazolyl, benzoxazolyl, benzisoxazolyl, benzothiazolyl, isobenzofuranyl, isoindolyl, purinyl, naphthyl, chinolinyl, chinoxalinyl and chinazolinyl.

8. The method according to any one of claims 1 to 7, wherein each of the aryl groups Ar¹ and Ar² of the boron- or silicon-containing aryl compounds and the aryldiazonium compound, respectively, comprises one or more substituents which are independently selected from the group consisting of hydrogen, halogen, nitro, hydroxy, cyano, carboxyl, C₁-C₆ carboxylic acid ester, C₁-C₆ ether, C₁-C₆ aldehyde, C₁-C₆ ketone, sulfonyl, C₁-C₆ alkylsulfonyl, C₁-C₆ alkyl, C₁-C₆ haloalkyl, C₁-C₈ cycloalkyl, C₁-C₈ halocycloalkyl (such as difluorocyclobutyl), C₁-C₈ heterocycloalkyl, C₁-C₆ alkoxy, C₁-C₆ haloalkoxy, C₃-C₁₂ aryl, C₃-C₁₂ heteroaryl and spiro-groups..

9. The method according to any one of claims 1 to 8, wherein the gold(I) catalyst is selected from the group consisting of (4-CF₃-C₆H₄)₃PAuCl, Ph₃PAuNTf₂, Cy₃PAuCl, (4-Me-C₆H₄)₃PAuCl and (4-CF₃-C₆H₄)₃PAuTf₂.

10. The method according to any one of claims 1 to 9, wherein the solvent is selected from the group consisting of MeOH, EtOH, MeCN.

11. The method according to any one of claims 1 to 10, wherein the method is carried out in the absence of a photosensitizer and external oxidant.

12. The method according to any one of claims 1 to 11, wherein irradiation in step (b) is carried out at a temperature of 0 to 60°C for a duration of 10 min. to 24 hours.

13. Use of a boron-containing compound represented by the following Formulae (i-1-1) to (i-4-1): 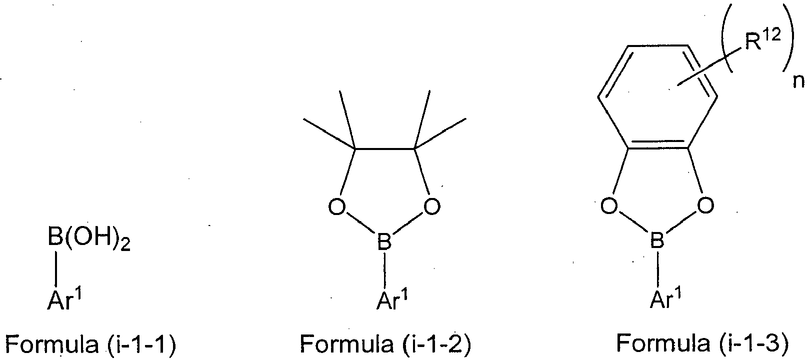 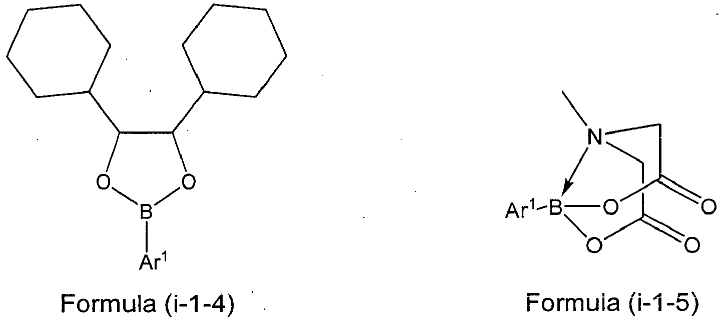 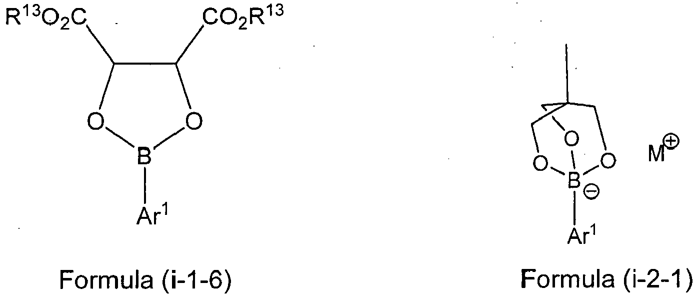 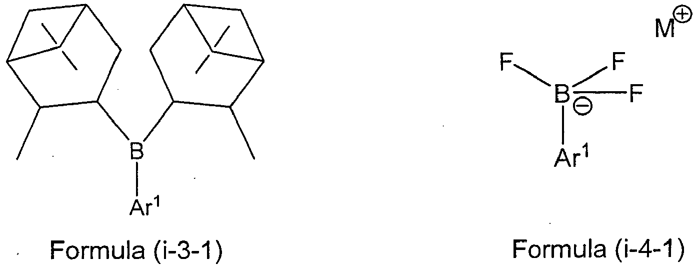 wherein
Ar¹, R¹², R¹³, n and M are defined above,
in the manufacture of functionalized biaryls.

14. Use of a silicon-containing compound represented by the following Formulae (i-1-1) to (i-4-1): 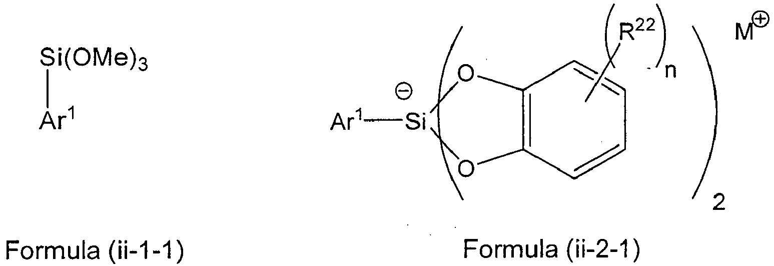 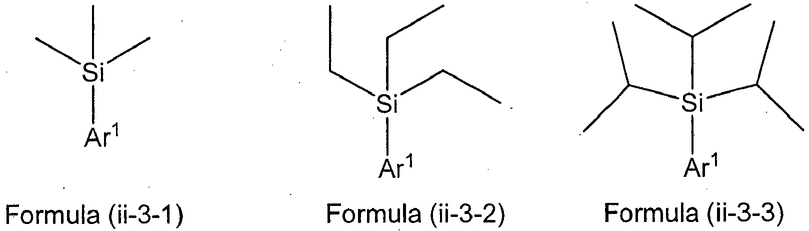 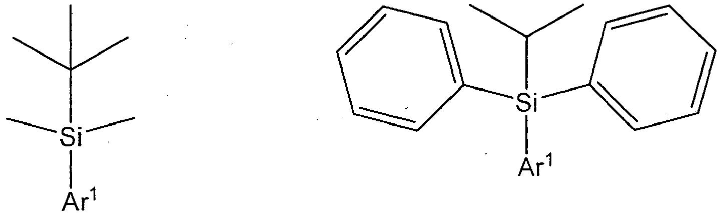 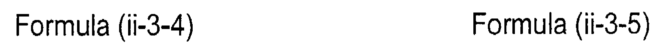 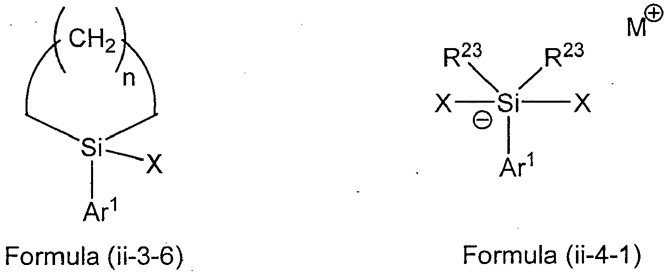 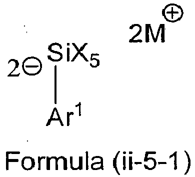 wherein
Ar¹, R²², R²³, X, n and M are defined above,
in the manufacture of functionalized biaryls.

15. Use of (4-CF₃-C₆H₄)₃PAuCl, Ph₃PAuNTf₂, Cy₃PAuCl, (4-Me-C₆H₄)₃PAuCl or (4-CF₃-C₆H₄)₃PAuTf₂ as a catalyst in the manufacture of optionally functionalized biaryls.
